# EUROPEAN PATENT APPLICATION

(11) **EP 4 710 966 A1**
(43) Date of publication of application: **18.03.2026**
(21) Application number: 24199805.3
(22) Date of filing: 11.09.2024
(51) Int. Cl.: A61M 5/20, A61M 5/315, A61M 5/32, A61M 5/24

(54) **AUTOINJECTOR AND METHOD OF RECONSTITUTING AN AUTOINJECTOR**

(71) Applicant: medmix Switzerland AG, 9469 Haag (Rheintal) (CH)
(72) Inventor: Jones, Matthew Meredith, Warwick, CV34 4AB (GB); Morris, Anthony Paul, Warwick, CV34 4AB (GB); Martin, Jack Gerald, Warwick, CV34 4AB (GB)
(74) Representative: Manitz Finsterwald Patent- und Rechtsanwaltspartnerschaft mbB

(57) **Abstract**

The present invention relates to an autoinjector comprising a dual chamber component comprising first and second chambers for storing first and second elements of a medicament that can each be stored in the respective first and second chambers of the dual chamber component, a piston rod, an inner body and an outer body, wherein the inner body and the outer body are in engagement with one another in a first configuration in a storage state and, on activation of the autoinjector are moved towards one another and then fixedly clipped to one another in a second configuration.

## Description

The present invention relates to an autoinjector comprising a dual chamber component comprising first and second chambers for storing first and second elements of a medicament that can each be stored in the respective first and second chambers of the dual chamber component, a piston rod, an inner body and an outer body, wherein the inner body and the outer body are in engagement with one another in a first configuration in a storage state and, on activation of the autoinjector are moved towards one another and then fixedly clipped to one another in a second configuration.

In various fields of applications such as for example the industrial, the medical and/or the dental sector it is common practice to provide dispensers or injectors that are capable of storing two or more components separately from one another and of mixing said components prior to said components being dispensed.

It has been found that this approach can also be favourable in the field of (self)application of certain medicaments by medical amateurs as certain medicaments consisting of two or more components can have an increased shelf life if stored separate from one another. Thereby the storage time for such medicaments can be enhanced drastically when the respective components are stored separately from one another such that they cannot interact with each other.

The crux with medical amateurs is that overcoming the bar for self-administering a medicament is often not easy. This can often lead to errors in application or even that sterility is broken which consequently then leads to even bigger issues.

This issue can be solved by providing (auto)injectors that are already prefilled with the right amount of each component of a certain medicament and that are configured to mix said components together before said mixture is then (self)injected by the user.

However, contrary to the dispensers used in the industrial sector for instance, it is necessary for (auto)injectors being used for medicaments to provide a sterile environment where the two or more components can be mixed with one another before the dispense of the mixed medicament as well as a sterile environment for the cannula with which said medicament is supposed to be injected.

In view of the above it is an object of the invention to provide an autoinjector with which all of the above issues can be overcome.

This object is satisfied by an autoinjector in accordance with the respective independent claim, such an autoinjector may comprise a dual chamber component comprising first and second chambers for storing first and second elements of a medicament that can each be stored in the respective first and second chambers of the dual chamber component, a piston rod, an inner body and an outer body, wherein the inner body and the outer body are in engagement with one another in a first configuration in a storage state and, on activation of the autoinjector are moved towards one another and then fixedly clipped to one another in a second configuration.

In this connection it should be noted that a dual chamber component is a component having two separate chambers with a respective chamber being provided for a respective part of a medicament with each part having a longer storage life than the combined and mixed medicament.

By way of example such a dual chamber component can be a dual chamber cartridge comprising first and second stoppers separating the two chambers of the cartridge from one another and to which a needle assembly can be connected.

Such a dual chamber cartridge may comprise a proximal and a distal stopper that are arranged one after another along a longitudinal axis of the dual chamber cartridge.

In this connection the inner body and the outer body surround internal components of the autoinjector and relative to which one or more of the internal components, such as the spring chassis, a needle guard and the piston rod can move and other components, such as the dual chamber component are held fixed in position.

The autoinjector may further comprise a spring chassis that is arranged moveable in the the outer body and the inner body on transferring the autoinjector from the second configuration to the third configuration. The spring chassis may be held fixed in position on transferring the autoinjector from the first configuration to the second configuration. The spring chassis is configured to be moved with respect to the body during a medicament dispensing process. It can thereby be driven by a spring acting thereon. The spring chassis is configured to entrain the piston rod such that the piston rod can engage one or more stoppers of the dual chamber component during the dispensing process respectively a dispensing operation.

During an activation process, also known as a medicament mixing or reconstitution process, the spring chassis is held in position with respect to the outer body, whereas the spring chassis is moved with respect to the inner body and the dual chamber component.

The inner body and the outer body may be clipped to one another in the first configuration, in this way the autoinjector can be protected against accidental activation.

The inner body and the outer body may be resting with respect to one another in the first configuration, in this way.

On moving the inner body towards the outer body, the piston rod may be moved into the dual chamber component for a mixing of medicament on the transition from the first configuration into the second configuration. In this way an activation process can be initiated in a simple manner.

On dispensing a medicament, the autoinjector is transferred from the second configuration into a third configuration. By way of example this autoinjector may be locked-out in the third configuration meaning that it can no longer be used to dispense medicament therefrom.

The autoinjector may further comprise a spring chassis, with the spring chassis being configured to be non-moveable held in the first configuration in at least one of the inner body and the outer body and with the spring chassis being configured to be moved proximally relative to both the inner body and the outer body once the autoinjector is transferred from the second configuration into the third configuration.

The autoinjector may further comprise a needle guard, wherein a proximal movement of said spring chassis is initiated by a distal movement of the needle guard relative to the body. Such a needle guard can beneficially be used to protect a person from becoming accidentally stabbed by the cannula or needle of the autoinjector after dispense.

Moreover, a proximal movement of said spring chassis may be initiated by a distal movement of said needle guard. Such an assembly minimizes construction size of the autoinjector.

The spring chassis may be moved in the autoinjector for a dispensing operation of the autoinjector.

The piston rod may be biased with respect to the spring chassis by a first biasing member. Thereby the piston rod can be moveable with respect to the spring chassis.

The spring chassis may be biased with respect to the outer body by a second biasing member, in particular wherein the spring chassis may be biased within the autoinjector during the first and second configurations with respect to said second biasing member, especially wherein the spring chassis is fixed within the autoinjector with respect to the spring bias of the second biasing member in the second configuration. Such a biasing member can bring about a movement of the spring chassis once desired.

A force of the second biasing member may be greater than a force of the first biasing member. In this way the second biasing member can cause the spring chassis to move relative to the piston rod and together with the piston rod relative to the dual chamber component.

The first biasing member may be a first compression spring, in particular wherein the first compression spring may have a spring force selected in the range of 3 to 5 N. Such a spring is simple to manufacture and to include in an autoinjector. Moreover, such a force is sufficient to ensure a movement of the distal stopper within the dual chamber component.

The second biasing member may be a second compression spring, in particular wherein the second compression spring has a spring force selected in the range of 5 to 40 N. Such a spring is simple to manufacture and to include in an autoinjector. Moreover, such a force is sufficient to ensure a movement of the piston rod through the dual chamber component.

The piston rod may be arranged within the spring chassis and moveable relative thereto as well as being entrainable by the spring chassis. In such a way a construction size of the autoinjector can be minimized.

The first biasing member may be arranged partly about the piston rod and at least partly within the spring chassis. In such a way a construction size of the autoinjector can be minimized.

In this connection it should be noted that the piston rod may be configured as a tube outside of a shaft of the spring chassis, with the first biasing member then being arranged within the tube and about the shaft of the spring chassis.

When the spring chassis is initially moved relative to the inner body and the outer body on transferring the autoinjector from the second configuration into the third configuration, the spring chassis may be moved relative to the piston rod until the spring chassis has compressed the first biasing member in order to entrain the piston rod together with the spring chassis for a dispensing operation. Such a movement, also known as over travel, allows the spring chassis to complete its minimum required displacement, even for low fill variants.

The autoinjector may further comprise a cap assembly having an outer cap and wherein a dispensing operation, i.e. transferred from the second configuration to the third configuration, of the autoinjector can only take place once the outer cap is removed. In this way the autoinjector can be protected from accidentally dispensing medicament.

The cap assembly may further have an inner cap and wherein the autoinjector can only be moved from the first configuration into the second configuration after removal of the inner cap from the autoinjector. In this way the autoinjector can be protected from accidentally being activated and contaminating the medicament stored therein.

The dual chamber component may comprise a cannula covered by a needle shield and the inner cap is configured to hold the needle shield. In this way a construction size can be reduced while ensuring efficient use of the autoinjector.

The needle shield may be removed from the autoinjector on removal of the inner cap. In this way a construction size can be reduced while ensuring efficient use of the autoinjector.

The first biasing member can be compressed during a dispensing operation, with a compression length of the first biasing member corresponding to a distance of travel of the spring chassis with respect to the piston rod on transferring the autoinjector from the second configuration to the third configuration, in particular wherein the compression length can be selected in a range of 0 to 40 mm, especially of 8 to 19 mm, preferably of 10 to 30 mm, especially wherein, once the spring chassis has travelled the compression length with respect to the piston rod, the spring chassis is configured to entrain the piston rod.

According to a further aspect there is provided a further autoinjector, such an autoinjector may comprise a housing, a spring chassis and a piston rod, wherein the piston rod is moveably arranged within the spring chassis and biased with respect to the spring chassis by means of a first biasing member, wherein the spring chassis is biased with respect to the housing by means of a second biasing member, and wherein a force of the second biasing member is greater than a force of the first biasing member.

In this way an autoinjector can be provided where the second biasing member can cause the spring chassis to move relative to the piston rod and together with the piston rod relative to the dual chamber component on dispensing medicament therefrom.

The first biasing member can be compressed during the dispensing operation, with a compression length of the first biasing member corresponding to a distance of travel of the spring chassis with respect to the piston rod on transferring the autoinjector from the second configuration to the third configuration.

The compression length can be selected in a range of 0 to 40 mm, especially of 8 to 19 mm, preferably of 10 to 30 mm. Such a compression length can be adapted in view of a dose to be dispensed.

Once the spring chassis has travelled the compression length with respect to the piston rod, the spring chassis may be configured to entrain the piston rod. Thereby the spring chassis can so to say over travel with respect to the piston rod.

A distance of travel of the spring chassis on a transfer of the autoinjector from the second configuration into the third configuration may be longer than a distance of travel of the piston rod. This is particularly beneficial for small dosages.

The longer distance of travel of the spring chassis with respect to the piston rod may at least essentially correspond to the compression length or corresponds to the compression length.

The outer body may comprise first and second snap fit windows and the inner body comprises a snap fit projection, wherein the snap fit projection engages the first snap window in the first configuration and the snap fit projection engages the second snap window in the second configuration. Such snap fit connections can reliably hold the outer body relative to the inner body during operation of the autoinjector.

The first and second snap fit windows may be linearly spaced apart from one another in an outer side surface of the outer body. In this way a construction size and complexity can be reduced.

The snap fit projection may comprise a tapered surface tapering outwardly form a distal end to a proximal end of the autoinjector. Such a tapered surface ensures a movement between the first and second snap windows.

The autoinjector may further comprise a catch assembly at a rear end of the piston rod. Such a catch assembly can aid in attaching the piston rod to the spring chassis while at the same time ensure that these components do not become disengaged.

The catch assembly may comprise at least one arm inclined with respect to the main extent of the piston rod. Such at least one arm can be used during assembly and also for ensuing maintained attachment.

According to a further aspect the present invention relates to a method of activating an autoinjector, in particular an autoinjector in accordance with one of the preceding claims, the autoinjector comprising an inner cap, an outer cap, an inner body, and an outer body, the method comprising the steps of: removing said inner cap from a proximal end of said autoinjector;
pressing the outer cap to move the inner body relative to the outer body; and
fixing said inner body to said outer body.

According to a further aspect of the invention the autoinjector according to the invention comprises a dual chamber cartridge comprising two elements of a medicament that are each stored in a separate chamber of the dual chamber cartridge. Said dual chamber cartridge also comprises a bypass passage. The autoinjector further comprises at least one security mechanism configured to secure the autoinjector against premature mixing of the elements and dispensing of said mixed elements.

That is, according to the invention an autoinjector is provided that comprises a dual chamber cartridge for two elements of a medicament that is configured to store each element separately from one another. Furthermore, said dual chamber cartridge comprises a bypass passage that may be configured to enable the two elements to be mixed with one another when a mixing mechanism is triggered.

Furthermore, the autoinjector according to the invention comprises at least one security mechanism that is configured to secure the autoinjector against premature mixing of the elements and dispensing of said mixed elements. In this connection it should further be noted that the security mechanism according to the invention can further be configured to provide a sterile environment for the elements, and in particular a cannula of said autoinjector, prior to mixing the elements respectively dispensing said mixture, i.e. injecting the medicament into a patient. The security mechanism may additionally be configured to protect a user from being injured by the autoinjector by for example shielding a cannula from the environment when the autoinjector is in a storage state.

Hence, with the autoinjector according to the invention a medical amateur can easily mix and (self)apply a medicament that consists of two or more elements as the chambers of the autoinjector are prefilled with the right dosage of elements. Furthermore, by providing a security mechanism, the autoinjector according to the invention is safely secured against premature mixing and dispensing such that the storage time of the elements contained in the autoinjector can be enhanced as they cannot come in contact with one another before a mixing mechanism is triggered. Additionally, said security mechanism can be configured to provide a sterile environment for all parts of the autoinjector that are involved in the injection process of the mixed medicament.

According to a further aspect of the invention, which may in particular be combined with the first aspect of the invention, an autoinjector is provided comprising a dual chamber cartridge comprising two elements of a medicament that are each stored in a separate chamber of the dual chamber cartridge and the dual chamber cartridge comprising a bypass passage. The dual chamber cartridge further comprises a cap and seal assembly covering and fixed to an outlet from the dual chamber cartridge.

That is, according to this further aspect of the invention the autoinjector comprises a dual chamber cartridge comprising two chambers configured to store two elements of a medicament, i.e. one medicament element in each chamber. Furthermore, the autoinjector comprises a bypass passage that is configured to enable the two elements to be mixed with one another when a mixing mechanism is triggered by for example allowing one element to flow from its storage chamber into the other chamber.

Furthermore, the autoinjector according to the invention comprises a cap and seal assembly covering and fixed to an outlet from the dual chamber cartridge. Said cap and seal assembly is therefore configured to close an outlet of the cartridge such that the elements stored therein can be held in a sterile environment. Furthermore, the cap and seal assembly can be configured to prevent a premature mixing and dispensing of said elements.

In this connection it may be noted that the cap and seal assembly may comprise a cap and one or more seals surrounded by the cap. Hence, according to this embodiment the cap and seal assembly can be a two-step assembly that ensures safe sealing of the dual chamber cartridge at least as long as it is in its storage state.

According to another embodiment of the invention the dual chamber cartridge has an opening which is covered by the cap and sealed off by one of said one or more seals of the cap and seal assembly. That is, the one or more seals can for example be configured to directly close the opening of the dual chamber cartridge while the cap covering said one or more seals may be configured to not only cover the outlet of the dual chamber cartridge but also said seal. The cap may in particular further be configured to (completely) surround the opening as well as the one or more seals.

According to a further embodiment the cap and seal assembly comprises a first flange arrangement and the dual chamber cartridge comprises a second flange arrangement shaped complementary to the first flange arrangement and being engageable by the first flange arrangement. Hence, said first and second flange arrangements may enable a secure engagement between the cap and seal assembly and the dual chamber cartridge.

In this connection it should be noted that the first flange arrangement can surround said second flange arrangement. It may in particular be possible that said first flange arrangement completely surrounds said second flange arrangement such that the first flange arrangement can generally be put on said second flange arrangement to provide a safe connection between the two.

It may further be possible that the first flange arrangement is snap-fit into place at the second flange arrangement. Such a snap fit connection can ensure a safe connection between the first and the second flange arrangement. It may in particular even provide a releasable connection which can potentially be useful in case the autoinjector should be reusable or in case the different parts of the autoinjector are supposed to be discarded separately from one another.

According to another embodiment of the invention the autoinjector further comprises a cannula held in place at the dual chamber cartridge by the cap and seal assembly. Said cannula may for example be configured as an injection needle. That is, said cannula may extend from an outside of the dual chamber cartridge to an inside of the dual chamber cartridge by extending beyond said cap and/or said one or more seals of the cap and seal assembly.

In this connection it is noted that the cannula may be held in place at the dual chamber cartridge by one of said one or more seals of the cap and seal assembly. That is, said one or more seals can further be configured to hold said cannula in place.

It may further be possible that the cap and seal assembly further comprises a cannula shield covering the cap and seal assembly. Such a cannula shield can ensure safe handling of the autoinjector as it prevents a user from being unintentionally stuck by said cannula. Additionally, said cannula shield can ensure a sterile environment for the outlet of the autoinjector, respectively said cannula, before using it on a patient as the outlet respectively the cannula is protected from the environment.

According to a further embodiment of the invention the dual chamber cartridge further comprises a proximal and a distal stopper. Said stopper may for example be configured to seal the two chambers of the dual chamber cartridge at at least one end of each chamber. Additionally or alternatively they may be configured to push the elements stored in said respective chambers towards the outlet of the autoinjector upon mixing and/or dispensing of the elements.

According to another embodiment the dual chamber cartridge comprises a first chamber more proximally arranged than a second chamber, wherein the first chamber is filled with a solid material and the second chamber is filled with a liquid material. That is, each element of the medicament can be stored in a separate chamber until they are intnetion-ally mixed with one another in order to enhance the storage time of said elements.

In this connection it should be noted that the first chamber may be separated from the second chamber by the proximal stopper. According to this embodiment no further separation or chamber walls are necessary to separate one chamber from the other as the proximal stopper can act as such a separation wall.

It may further be possible that the proximal stopper is arranged distally of the bypass passage in a storage state of the autoinjector. This prevents the element stored in the second chamber from reaching the first chamber as the bypass passage is blocked by the proximal stopper.

In this connection it should further be noted that the proximal stopper can be arranged proximally of the bypass passage after a dispensing operation has taken place. That is, the proximal stopper may be arranged moveably in the autoinjector such that it may first be arranged distally of the bypass passage in a storage state and then proximally of the bypass passage after a dispensing operation has taken place.

By having the proximal stopper arranged proximally of the bypass passage after a dispensing operation has taken place it can further be prevented that any residuals of the medicament left in the first chamber can flow back into the second chamber behind the proximal stopper. This can be particularly important in applications where not all of the mixed medicament is injected at once but rather by two or more injections throughout a predetermined period of time.

According to a further embodiment the autoinjector further comprises a piston rod configured to act on said distal stopper and said proximal stopper and to thereby move the proximal stopper such that it is arranged at the bypass passage for urging the material into the proximal chamber past the proximal stopper via the bypass passage during a mixing process.

That is, according to this embodiment both the distal and the proximal stopper are arranged moveably in the dual chamber cartridge to be able to urge the elements that are arranged right in front of the respective stoppers towards the outlet of the dual chamber cartridge.

In this connection it should be noted that as the element that is stored in the distal chamber is urged through the bypass passage into the proximal chamber during the mixing process, it may be possible for the distal and the proximal stopper to come into contact to later move together as one.

It may thus be possible that the piston rod can be moved further for dispensing the medicament via the cap and seal assembly. That is, the piston rod can be moved such that it brings both stoppers towards the cap and seal assembly to urge the mixed material towards the outlet.

According to a further embodiment the one of said one or more seals of the cap and seal assembly sealing off the opening covers said opening. By sealing the opening it can for example be ensured that the mixed medicament can only be dispensed via the cap and seal assembly and/or a cannula.

According to yet another embodiment the autoinjector further comprises a cannula shield such as a rigid cannula shield covering a cannula of the dual chamber cartridge. Such a cannula shield can for example be provided to ensure safe handling of the autoinjector and to prevent injuries by shielding the user from the cannula. Furthermore, said cannula shield can be configured to provide a sterile environment for the cannula before the first use of the autoinjector.

According to a further aspect of the invention a method of mixing a medicament in an autoinjector is provided with the autoinjector comprising a dual chamber cartridge comprising first and second elements of a medicament, with the first and second elements each being stored in a separate chamber of the dual chamber cartridge, the dual chamber cartridge comprising a cap and seal assembly as well as a piston rod. The method according to the invention comprises the steps of:
- moving the piston rod proximally towards a distal stopper and thereby moving said distal stopper towards a proximal stopper and reducing the distance between the distal stopper and said proximal stopper to thereby urge a first element of the medicament from a first chamber into a bypass passage to bypass the proximal stopper such that the first element comes into contact with the second element in the first chamber arranged between the cap and seal assembly and the proximal stopper.

That is, according to the invention it is possible to store two elements of a medicament separately in two different chambers in order to enhance the storage time of one or more of said elements.

Then, by moving the piston rod proximally towards the distal stopper such that said distal stopper is also guided towards the proximal stopper, the element that is stored in the distal chamber is urged towards said proximal stopper. When the pressure that acts on said proximal stopper is high enough, also the proximal stopper will start moving in a proximal direction thereby releasing the bypass channel such that the second element stored in the distal, i.e. the second, chamber can flow through said bypass channel to bypass the proximal stopper and to reach the proximal, i.e. the first, chamber where the first element is stored. This allows a mixing of the two elements.

It may additionally also be possible to move the piston rod further after the mixing of the elements is complete in order to urge the mixed medicament out of an outlet of the autoinjector in order to inject said medicament into a patient.

According to a further aspect of the invention an autoinjector comprising a dual chamber cartridge comprising two elements of a medicament that are each stored in a separate chamber of the dual chamber cartridge is provided. The dual chamber cartridge according to the invention comprises a bypass passage and a rubber septum and a cannula shield. The autoinjector further comprises a cannula carriage having a cannula with the cannula carriage being in contact with the dual chamber cartridge in a storage state of the autoinjector and the dual chamber cartridge being moveable towards the cannula carriage on activation of the autoinjector in order to penetrate the septum with the cannula.

That is, according to this further aspect of the invention the autoinjector comprises a dual chamber cartridge comprising two chambers configured to store two elements of a medicament, i.e. one element of medicament in each chamber.

Furthermore, the autoinjector comprises a bypass passage that may be configured to enable the two elements to be mixed with one another when a mixing mechanism and/or an activation mechanism is triggered.

According to this aspect of the invention the autoinjector further comprises a cannula carriage having a cannula with the cannula carriage being in contact with the dual chamber cartridge in a storage state. The cannula carriage may thus enable a safe connection of the cannula at the dual chamber cartridge.

However, in a storage state, said cannula carriage is only in contact with the dual chamber cartridge but the cannula itself does not yet extend into said dual chamber cartridge. This is only the case when the autoinjector is activated by moving the dual chamber cartridge towards the cannula carriage (or the other way around) such that the cannula can penetrate the septum of the dual chamber cartridge to extend beyond said septum into said dual chamber cartridge thereby providing a flow channel for the elements stored inside the autoinjector.

According to an embodiment of the invention a distal end of the cannula carriage is sealed off by a seal to make sure that the elements stored inside the dual chamber cartridge can only flow through said cannula.

In this connection it should be noted that said seal may be moveably arranged in a distal carriage reception space relative to the cannula. By arranging said seal moveable in said distal carriage reception space, the seal can move relative to the cannula upon activation of the autoinjector when the dual chamber cartridge is moved towards the cannula carriage. It may thus be possible that said seal moves from a distal end of the cannula carriage reception space towards a proximal end of said cannula carriage reception space upon activation of the autoinjector.

According to a further embodiment the cannula carriage comprises one or more detents at a distal end thereof. Said detents may be configured to hold the dual chamber cartridge, i.e. to secure the dual chamber cartridge at the cannula carriage.

It may further be possible that the cannula carriage is in contact with the dual chamber cartridge via the one or more detents in the storage state of the autoinjector. Said detents may for example be configured to secure the cannula carriage at the dual chamber cartridge. Additionally or alternatively, they may be configured to hold the cannula carriage at a predetermined distance with respect to the dual chamber cartridge.

Furthermore, on activation of the autoinjector, the dual chamber cartridge may be configured to overcome said one or more detents such that the cannula pierces the septum. That is, the one or more detents may be configured to provide a securing mechanism that has to be overcome upon activation of the autoinjector.

It could further be possible that a movement of the dual chamber cartridge towards the cannula carriage moves said seal towards the cannula to pierce said seal on activation of the autoinjector.

In this connection it should also be noted that the movement of the dual chamber cartridge towards the cannula carriage may move said seal towards the cannula to first pierce said seal and then secondly pierce said septum to allow the cannula to extend beyond said seal and said septum into the dual chamber cartridge.

According to another embodiment of the invention the autoinjector further comprises a distal stopper arranged in said dual chamber cartridge, and a piston rod, wherein, on activation of said autoinjector, a movement of said piston rod towards the distal stopper brings about a movement of said dual chamber cartridge towards said cannula carriage. That is, to activate the autoinjector a user may move the piston rod towards said distal stopper which then translates into a movement of the dual chamber cartridge towards the cannula carriage.

It may further be possible that once said septum is pierced, a movement of said distal stopper within the dual chamber cartridge is facilitated. Such a movement of the distal stopper may for example urge the elements stored in the dual chamber cartridge towards the cannula in order to be dispensed, i.e. injected into a patient.

According to another embodiment of the invention the dual chamber cartridge comprises a proximal and a distal stopper and a bypass passage. Said proximal and distal stoppers may have various tasks. For example, the distal stopper can be configured to be moved towards the proximal stopper upon activation of the autoinjector.

Furthermore, it may possible that the proximal stopper is configured to urge the elements that are stored in the autoinjector towards the cannula upon a dispensing motion of the autoinjector, in particular together with the distal stopper.

The proximal and the distal stopper may be arranged at a distance from one another in a storage state in order to provide two chambers in which the elements of the medicament can be stored separately from one another.

The bypass passage may be arranged at or near the proximal stopper such that upon activation of the autoinjector said proximal stopper may move towards said bypass passage such that the element stored behind, i.e. distally relative to the proximal stopper can flow through said bypass passage to bypass the proximal stopper and to reach the element that is stored in front of, i.e. proximally relative to the proximal stopper in order to be mixed with one another.

It may further be possible that the dual chamber cartridge comprises a first chamber more proximally arranged than a second chamber, wherein the first chamber is filled with a solid material of said medicament and the second chamber is filled with a liquid material of said medicament.

According to one embodiment the first chamber is separated from the second chamber by the proximal stopper. That is, in this case no additional separation walls or the like are necessary to separate the first chamber from the second chamber.

In this connection it should be noted that in some cases the autoinjector further comprises a piston rod configured to act on said distal stopper and said proximal stopper and to thereby move the proximal stopper such that it is arranged at the bypass passage for urging the liquid material into the first chamber past the proximal stopper via the bypass passage such that the liquid material can be mixed with the solid material.

According to a further aspect of the invention a method of activating an autoinjector is provided, the autoinjector comprising a dual chamber cartridge comprising first and second elements of a medicament, with the first and second elements each being stored in a separate chamber of the dual chamber cartridge and a cannula carriage having a cannula and a piston rod. The method according to the invention comprises the steps of:
- moving the piston rod proximally towards a distal stopper arranged in the dual chamber cartridge and thereby moving said dual chamber cartridge towards the cannula carriage to pierce a septum of said dual chamber cartridge with said cannula.

In some cases the method may further comprise the step of allowing air to be expelled from said dual chamber cartridge via said cannula and then further moving the distal stopper towards a proximal stopper to thereby urge a second element of the medicament from a second chamber into a bypass passage to bypass the proximal stopper such that the second element comes into contact with the first element in the first chamber arranged between the cannula carriage and the proximal stopper.

According to a further aspect of the invention an autoinjector comprising a dual chamber cartridge comprising two elements of a medicament that are each stored in a separate chamber of the dual chamber cartridge is provided. The dual chamber cartridge further comprises a bypass passage and an outer cap, an inner cap and a cannula shield covering a cannula of the autoinjector, wherein, on activation of the autoinjector said inner cap is removable from the autoinjector before said outer cap is removed from said autoinjector.

That is, according to this further aspect of the invention the autoinjector comprises a dual chamber cartridge comprising two chambers configured to store two elements of a medicament, i.e. one medicament in each chamber. Furthermore, the autoinjector comprises a bypass passage that may be configured to enable the two elements to be mixed with one another when a mixing mechanism is triggered.

The autoinjector according to the invention further comprises an outer cap, an inner cap and a cannula shield covering a cannula of the autoinjector. On activation of the autoinjector said inner cap is removable from the autoinjector before said outer cap is removed from said autoinjector.

Said inner and outer caps may have the same or different functions. For example they may both be configured to shield a user from the cannula when the autoinjector is in the storage state. Additionally or alternatively either one of the inner and the outer cap or both may be configured to keep the autoinjector in a storage state to prevent premature mixing and/or dispensing of the elements stored in the dual chamber cartridge.

It can for example be possible that the inner cap is configured to prevent premature mixing of the elements stored in the dual chamber cartridge while the outer cap is configured to prevent premature dispensing of the elements.

The cannula shield can further be configured to shield the cannula and/or to ensure a sterile environment for the cannula in the storage stage of the autoinjector.

Additionally, the cannula shield can also be configured to keep the autoinjector from prematurely mixing and dispensing the elements stored therein, for instance. In this connection it should be noted that it would also be possible to complete mixing and to accept a certain degree of over pressure in the proximal chamber of the syringe.

According to an embodiment of the invention the autoinjector is configured such that removal of the inner cap simultaneously brings about a removal of the cannula shield such that only one action is required from a user to remove both the inner cap and the cannula shield to start an activation of the autoinjector. In this connection it should be noted that the mixing process of the two materials should be avoided prior to removal of the inner cap, hence the reconstitution or mixing process is preferably hindered prior to removal of the inner cap, so that removal of the inner cap is considered a first stage of activation of the autoinjector.

In this connection it should be noted that the inner cap may comprise a grip projecting from the autoinjector to facilitate the removal of the inner cap for the user.

The grip may comprise a ring which can be easily gripped by a user.

According to another embodiment removal of the inner cap permits a movement of air out of the dual chamber cartridge. This may facilitate the mixing of the two elements stored in the dual chamber cartridge.

According to another embodiment of the invention the inner cap comprises a cannula shield holder at an end of the inner cap disposed opposite to a base of the inner cap such that the inner cap may be configured to additionally hold a cannula shield.

According to a further embodiment the inner cap comprises inwardly facing projections that engage the cannula shield. This may for example be advantageous in case the cannula shield is supposed to be removed together with the inner cap as said projections can safely secure the inner cap to the cannula shield.

Said inwardly facing projections may for example be integrally formed with the cannula shield holder of the inner cap. This can lead to lower production costs and time as less single components are needed to form the inner cap, the inwardly facing projections and the cannula shield holder. Thus, one could even think of providing an inner cap, cannula shield holder and inwardly facing projections that are made as one-piece unit. Alternatively, only the cannula shield holder and the projections could be made as a single unit, for instance.

In this connection it should further be noted that the inwardly facing projections may also be formed by a metal insert received within the cannula shield holder of the inner cap which may have benefits in view of durability and strength of the inwardly facing projections.

According to a further embodiment the outer cap is configured to receive a part of a cannula shield of the autoinjector.

According to yet another embodiment an axial movement of a cannula guard of the autoinjector in the direction of the dual chamber cartridge brings about a release of a drive mechanism of a plunger of the dual chamber cartridge for dispensing a medicament stored in the dual chamber cartridge.

The devices and mechanisms according to the present disclosure may be configured to store and subsequently dispense a drug or, used synonymously herein, a medicament.

According to the present disclosure, a drug or a medicament is a pharmaceutical formulation that contains one or more active pharmaceutical ingredients. An active pharmaceutical ingredient is a substance that is biologically active or has a biological effect on humans or animals. It is used in the diagnosis, mitigation, cure, treatment or prevention of a medical condition or to otherwise affect the structure or function of the body of humans or animals.

Active pharmaceutical ingredients, medicaments and drugs that may be contained and/or dispensed by the mechanisms and devices according to the present disclosure are, inter alia, described in handbooks such as Merck Index, for example 15th edition, Rote Liste, such as Rote Liste 2023, the publication Approved Drug Products with Therapeutic Equivalence Evaluations (Orange Book) by the U.S. FDA and the Purple Book Database of Licensed Biological Products, also by the U.S. FDA, all of which are freely accessible via the Internet.

Active pharmaceutical ingredients, medicaments and drugs that may be contained and/or dispensed by the mechanisms and devices according to the present disclosure may be selected from: analgesics and antirheumatics (main group 05 of Rote Liste); anti-obesity drugs (main group 06 of Rote Liste); antiallergics (main groups 07 of Rote Liste); antiane-mics (main group 08 of Rote Liste); antibiotics (main group 10 of Rote Liste); antidiabetic drugs (main group 12 of Rote Liste); antidote medications (main group 13 of Rote Liste); antihemorrhagics (antifibrinolytics and other hemostatic agents) (main group 16 of Rote Liste); anti hypoglycemic drugs (main group 18 of Rote Liste); antihypotonics and agents for shock therapy (main group 19 of Rote Liste); anticoagulants (main group 20 of Rote Liste); beta-receptor blockers, calcium channel blockers and inhibitors of the renin-angiotensin-aldosterone system (main group 27 of Rote Liste); bronchospasmolytics/antiasthmatics and other agents for the respiratory tract (main group 28 of Rote Liste); dermatics (main group 32 of Rote Liste); diagnostic agents and agents for diagnostic preparation (main group 35 of Rote Liste); enzyme inhibitors, preparations for enzyme deficiency and transport proteins (main group 40 of Rote Liste); gene and cell therapeutics and RNA interference therapeutics (main group 42 of Rote Liste); hypophysial and hypothalamic hormones, other regulatory peptides, their inhibitors and analogues (main group 50 of Rote Liste); immunomodulators (main group 51 of Rote Liste); lipid-lowering drugs (main group 58 of Rote Liste); migraine medication (main group 61 of Rote Liste); neuropathy preparations and other neurotropic agents (main group 66 of Rote Liste); ophthalmics (main group 67 of Rote Liste); osteoporosis agents, calcium/bone metabolism regulators (main group 68 of Rote Liste); psychotropic drugs (main group 71 of Rote Liste); sera, immunoglobulins and vaccines (main group 75 of Rote Liste); sex hormones and their inhibitors (main group 76 of Rote Liste); vitamins (main group 84 of Rote Liste); oncology drugs, cytostat-ics, other antineoplastic agents and protectives (main group 86 of Rote Liste); anaesthetics; autoimmune disorder medication, such as rheumatoid arthritis medication and/or multiple sclerosis medication; and/or cardiovascular disease medication.

In a specific example, an active ingredient of a medicament delivered by the mechanisms and devices according to the present disclosure is an antirheumatic.

In a further specific example, an active ingredient of a medicament delivered by the mechanisms and devices according to the present disclosure is an anti-obesity drug.

In a further specific example, an active ingredient of a medicament delivered by the mechanisms and devices according to the present disclosure is an antiallergic.

In a further specific example, an active ingredient of a medicament delivered by the mechanisms and devices according to the present disclosure is an antibiotic.

In a further specific example, an active ingredient of a medicament delivered by the mechanisms and devices according to the present disclosure is an antidiabetic drug.

In a further specific example, an active ingredient of a medicament delivered by the mechanisms and devices according to the present disclosure is an antihypoglycemic drug.

In a further specific example, an active ingredient of a medicament delivered by the mechanisms and devices according to the present disclosure is an enzyme inhibitor. In a further specific example, an active ingredient of a medicament delivered by the mechanisms and devices according to the present disclosure is a preparation for enzyme deficiency. In a further specific example, an active ingredient of a medicament delivered by the mechanisms and devices according to the present disclosure is a transport protein.

In a further specific example, an active ingredient of a medicament delivered by the mechanisms and devices according to the present disclosure is a gene therapeutic. In a further specific example, an active ingredient of a medicament delivered by the mechanisms and devices according to the present disclosure is a cell therapeutic. In a further specific example, an active ingredient of a medicament delivered by the mechanisms and devices according to the present disclosure is an RNA interference therapeutics.

In a further specific example, an active ingredient of a medicament delivered by the mechanisms and devices according to the present disclosure is a hypophysial hormone. In a further specific example, an active ingredient of a medicament delivered by the mechanisms and devices according to the present disclosure is a hypothalamic hormone. In a further specific example, an active ingredient of a medicament delivered by the mechanisms and devices according to the present disclosure is a regulatory peptide other than a hypophysial hormone or a hypothalamic hormone.

In a further specific example, an active ingredient of a medicament delivered by the mechanisms and devices according to the present disclosure is an inhibitor of a hypophysial hormone. In a further specific example, an active ingredient of a medicament delivered by the mechanisms and devices according to the present disclosure is an inhibitor of a hypothalamic hormone. In a further specific example, an active ingredient of a medicament delivered by the mechanisms and devices according to the present disclosure is an inhibitor of a regulatory peptide other than a hypophysial hormone or a hypothalamic hormone.

In a further specific example, an active ingredient of a medicament delivered by the mechanisms and devices according to the present disclosure is an analogue of a hypophysial hormone. In a further specific example, an active ingredient of a medicament delivered by the mechanisms and devices according to the present disclosure is an analogue of a hypothalamic hormone. In a further specific example, an active ingredient of a medicament delivered by the mechanisms and devices according to the present disclosure is an analogue of a regulatory peptide other than a hypophysial hormone or a hypothalamic hormone.

In a further specific example, an active ingredient of a medicament delivered by the mechanisms and devices according to the present disclosure is an immunomodulator.

In a further specific example, an active ingredient of a medicament delivered by the mechanisms and devices according to the present disclosure is a lipid-lowering drug.

In a further specific example, an active ingredient of a medicament delivered by the mechanisms and devices according to the present disclosure is an osteoporosis agent.

In a further specific example, an active ingredient of a medicament delivered by the mechanisms and devices according to the present disclosure is a calcium metabolism regulator.

In a further specific example, an active ingredient of a medicament delivered by the mechanisms and devices according to the present disclosure is a bone metabolism regulator.

In a further specific example, an active ingredient of a medicament delivered by the mechanisms and devices according to the present disclosure is a sex hormone.

In a further specific example, an active ingredient of a medicament delivered by the mechanisms and devices according to the present disclosure is an inhibitor of a sex hormone.

In a further specific example, an active ingredient of a medicament delivered by the mechanisms and devices according to the present disclosure is an autoimmune disorder medication.

In a further specific example, an active ingredient of a medicament delivered by the mechanisms and devices according to the present disclosure is a rheumatoid arthritis medication.

In a further specific example, an active ingredient of a medicament delivered by the mechanisms and devices according to the present disclosure is a multiple sclerosis medication.

In a further specific example, an active ingredient of a medicament delivered by the mechanisms and devices according to the present disclosure is a cardiovascular disease medication.

The drug or medicament may have, for example, one or more active pharmaceutical ingredients selected from one or more of: carbohydrates and polysaccharides; polypeptides, peptides and proteins (e.g., antibodies, antibody fragments, hormones, growth factors, and enzymes); small molecules with a molecular weight of 500 Da or less; and nucleic acids, double or single stranded DNA (including cDNA and naked DNA), RNA, antisense nucleic acids, such as antisense DNA and RNA, small interfering RNA (siRNA), ribozymes, genes, and oligonucleotides. Nucleic acids may be included into molecular delivery systems such as liposomes, vectors, or plasmids.

The drugs or medicaments contained in or dispensed with the mechanisms according to the present disclosure may comprise active pharmaceutical ingredients used in the treatment and/or mitigation and/or prevention and/or cure and/or diagnosis of many different types of medical conditions. These may include, inter alia, one or more of the following conditions: acute coronary syndrome, angina, myocardial infarction, anaphylactic shock, atherosclerosis, diabetes mellitus or complications associated with type 1 or type 2 diabetes mellitus such as diabetic retinopathy, cancer, macular degeneration, inflammation, hay fever, rheumatoid arthritis, thromboembolism disorders such as deep vein or pulmonary thromboembolism, infertility, growth disorder, migraine, autoimmune disorders, multiple sclerosis, osteoporosis, allergies, obesity, and/or cardiovascular diseases.

With a specific example, the condition may be diabetes mellitus.

With another specific example, the condition may be a complication associated with type 1 diabetes mellitus. With another specific example, the condition may be a complication associated with type 2 diabetes mellitus. With another specific example, the condition may be diabetic retinopathy.

With another specific example, the condition may be an inflammation.

With another specific example, the condition may be rheumatoid arthritis.

With another specific example, the condition may be infertility.

With another specific example, the condition may be growth disorder.

With another specific example, the condition may be migraine.

With another specific example, the condition may be an autoimmune disorder.

With another specific example, the condition may be multiple sclerosis.

With another specific example, the condition may be osteoporosis.

With another specific example, the condition may be an allergy.

With another specific example, the condition may be obesity.

With another specific example, the condition may be a cardiovascular disease.

For example, the active pharmaceutical ingredients for the therapy of type 1 or type 2 diabetes mellitus or complications associated with type 1 or type 2 diabetes mellitus include an insulin, such as, human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof.

The active pharmaceutical ingredient may also be a hormone, such as a hypophysis hormone or a hypothalamus hormone or a regulatory active peptide and an antagonist of a regulatory active peptide. The hormone may be a fertility hormone, a gonadotropine, a growth hormone, a steroid hormone, such as testosterone or oestrogen, a parathyroid hormone, such as teriparatide, epinephrine or a follicle-stimulating hormone.

The active pharmaceutical ingredient may also be an oligonucleotide.

The active pharmaceutical ingredient may also be an antibody. The term "antibody" includes an immunoglobulin molecule or an antigen-binding portion, such as a fragment antigen-binding region, of an immunoglobulin molecule, an antigen-binding molecule based on tetravalent bispecific tandem immunoglobulins, and/or a dual variable region antibody-like binding protein having cross-over binding region orientation (CODV). The antibody may be a monoclonal, a polyclonal, a recombinant, or a chimeric antibody. It may be a de-immunized or humanized, a fully human, or a non-human, such as a murine, antibody. The antibody may be a single chain antibody.

The active pharmaceutical ingredient may also be a polysaccharide, such as a glucosaminoglycane, a hyaluronic acid, a heparin, a low molecular weight heparin or an ultra-low molecular weight heparin or a derivative thereof. The active pharmaceutical ingredient may also be a sulphated polysaccharide, e.g. a poly-sulphated form of the above-mentioned polysaccharides, and/or a pharmaceutically acceptable salt thereof.

The drug or medicament contained in and/or dispensed with the mechanisms and devices according to the present disclosure may include one or more of the following active pharmaceutical ingredients: abatacept, adalimumab, aflibercept, alirocumab, anakinra, apomorphine, aripiprazole, belimumab, benralizumab, betamethasone, bimekizumab, brodalumab, brolucizumab, buprenorphine, certolizumab pegol, choriogonadotropin alfa, cyanocobalamin, carbepoetin alfa, denosumab, dulaglutide, dupilumab, enoxaparin sodium, epinephrine, epoetin, epoetin alfa, epoetin beta, epoetin theta, epoetin zeta, erenumab, etanercept, evolocumab, exenatide, filgrastim, follitropin, follitropin alfa, follitropin delta, folic acid, fondaparinux, fremanezumab, fulvestrant, gadobenic acid, gadobutrol, gadodiamide, gadoteric acid, gadoxetic acid, galcanezumab, ganirelix, glatiramer, glucagon, golimumab, goserelin, guselkumab, heparin, icatibant, inclisiran, infliximab, inotersen, insulin aspart, insulin degludec, insulin detemir, insulin glargine, insulin glulisine, insulin human, insulin human-isophane, insulin lispro, insulin isophane, interferon beta-1a, ixekizumab, lanadelumab, lanreotide, lebrikizumab, leuprorelin, liraglutide, lonapegsomatropin, lutropin alfa, menotropin, mepolizumab, methotrexate, methoxy-polyethylene glycol-epoetin beta, mirikizumab, moroctocog alfa, natalizumab, nirsevimab, ofatumumab, omalizumab, paliperidone, pegfilgrastim, peginterferon alfa-2a, peginterferon beta-1a, pegvaliase, propofol, pyridoxine, ranibizumab, rho(D) immune globulin, risankizumab, romosozumab, ropeginterferon alfa-2b, sarilumab, satralizumab, secukinumab, semaglutide, somapacitan, somatrogon, somatropin, sumatriptan, teriparatide, tezepelumab, tildrakizumab, tinzaparin sodium, tocilizumab, tralokinumab, triptorelin, ustekinumab, vedolizumab, volanesorsen, and vutrisiran.

Additionally or alternatively, the drug or medicament contained in and/or dispensed with the mechanisms and devices according to the present disclosure may include one or more of the following active ingredients: Lixisenatide, Taspoglutide, Albiglutide, Efpeglenatide, GLP-1 Eligen, Nodexen, Pegapamodtide, Tirzepatide, and Bamadutide.

Additionally or alternatively, the drug or medicament dispensed with the mechanisms and devices according to the present disclosure may include one or more of the following active ingredients: Mipomersen sodium, Linagliptin, Vildagliptin, Sitagliptin, Denagliptin, Saxagliptin, Berberine, Lutropin, Choriongonadotropin, Desmopressin, Terlipressin, Gonadorelin, Buserelin, Nafarelin, Hylan G-F 20.

Additionally or alternatively, the drug or medicament dispensed with the mechanisms and devices according to the present disclosure may include as an active ingredient a piperidine alkaloid, such as azimine, azithromycine, azcarpine, and/or carpaine.

The drug or medicament may also contain one or more of the following: pharmaceutically acceptable solvents or salts of the active pharmaceutical ingredients described herein; analogues and derivates of the active pharmaceutical ingredients described herein; biosimilars of the active pharmaceutical ingredients described herein; and pharmaceutically acceptable carriers of the active pharmaceutical ingredients described herein.

The drug or medicament contained in and/or dispensed with the mechanisms and devices according to the present disclosure may include one or more of the following active pharmaceutical ingredients: paracetamol (acetaminophen), nonsteroidal anti-inflammatory drugs, such as aspirin, ibuprofen and naproxen, cox-2 inhibitors, such as rofecoxib, celecoxib, and etoricoxib, opioids, such as morphine, codeine, oxycodone, hydrocodone, dihydromorphine, pethidine, alcohols, cannabis, nefopam, flupirtine, ziconotide.

The drug or medicament may be contained in a container from which it is dispensed by an action of a user. The container may, for example, be a cartridge, a reservoir or a syringe. The container may extend between a dispensing end and a rear end of the container. The container may be configured to dispense the drug or medicament at the dispensing end, for example through a pierceable septum. At the rear end, the container may be sealed to prevent the exit of the drug or medicament. For example, the container may be sealed by a flexible and/or displaceable plunger at the rear end. In between the dispensing end and the rear end, the container may have a generally tubular hollow housing.

The container may have a single compartment for storing the drug or medicament. Alternatively, the container may also have more than one compartment, such as two compartments. Different compartments thereby may comprise different substances, such as drugs, medicaments, active ingredients, solvents or carriers. Different compartments may also comprise a part of the medicament or drug in solid form, for example as a powder, such as a lyophilized powder, and another part of the medicament in liquid form. The solid part may, for example, comprise the one or more active pharmaceutical ingredient and/or the liquid part may be a solvent.

The container may be configured to allow a mixing of the contents from at least two of the compartments, such as from all of the compartments, prior to and/or during dispensing of the medicament or drug. For example, the container may be configured to provide a fluid connection between the at least two, such as between all of the compartments, for mixing.

For example, the container may comprise two compartments, one comprising a first part of the drug or medicament in solid form, for example the one or more active ingredients, and the other one comprising a second part of the drug or medicament in liquid form, such as a solvent. The container may be configured to provide a fluid connection between the two compartments and to mix the first and second parts prior or during dispensing. This may reconstitute the drug or medicament from its solid form.

The devices and mechanisms according to the present disclosure may be disposable, which means that the container holding the medicament is non-replaceable or non-refillable and the devices are disposed of after having dispensed a last dose from the container. Alternatively, they may be reusable, for example, they may allow to replace a container by a new container or to refill the container.

The devices and mechanisms according to the present disclosure may be fixed dose devices and mechanisms that only allow the dispensing of a fixed dose of the medicament or drug. Alternatively, they may be variable dose devices and mechanisms that allow for setting of a dose from a multitude of differing settable doses.

The mechanisms and devices according to the present disclosure may comprise a dose definition mechanism that allows a user to set the dose to be dispensed prior to dispensing. The dose thereby may be settable as a fixed dose only, for example for fixed dose devices or mechanisms. The dose also may be settable from a multitude of settable doses, such as for variable dose devices or mechanisms.

The doses may be settable as integer multiples of international units of the drug or medicament. They also may be settable as integer multiples of a fraction of said international units, such as integer multiples of half units.

The mechanisms and devices according to the present disclosure may be single dose devices that allow dispensing of a dose from the container only once, so that the container is a single dose container. They also may be multiple dose devices that allow for a sequential dispensing of more than one dose from the same container, so that the container is a multi-dose container.

The drug delivery devices according to the present disclosure may be injection devices, such as needle-based injection devices.

Features and functions that may be implemented in mechanisms and devices according to the present disclosure, and/or requirements that may be met by those mechanisms and devices are also described in standard ISO 11608-1 (Needle-based injection systems for medical use - Requirements and test methods - Part 1: Needle-based injection systems), such as ISO 11608-1:2022(E), and standard ISO 11608-5 (Needle-based injection systems for medical use - Requirements and test methods - Part 5: Automated functions), such as ISO 11608-5:2022(E).

The invention is further described by the following embodiments and Figures which show:
- Fig. 1:: various sectional views of different stages of use of a dual chamber autoinjector;
- Fig. 2: a sectional view of a pre-filled syringe including a cannula shield;
- Fig. 3:: a sectional view of the pre-filled syringe of Fig. 2, without the cannula shield;
- Fig. 4:: a sectional view of a dual chamber cartridge comprising a cannula carriage and a cannula shield;
- Fig. 5:: a sectional view of the dual chamber cartridge of Fig. 4 without the cannula shield;
- Fig. 6:: a close-up view of the cannula carriage of Figs. 4 and 5;
- Fig. 7:: detailed views of the outer and inner caps of the autoinjector;
- Fig. 8:: detailed views of a cap;
- Fig. 9:: detailed views of an inner body;
- Fig. 10:: detailed views of an outer body;
- Fig. 11:: detailed views of a piston rod;
- Fig. 12:: detailed views of a spring chassis;
- Fig. 13:: detailed views of a first kind of cannula;
- Fig. 14:: detailed views of a second kind of cannula

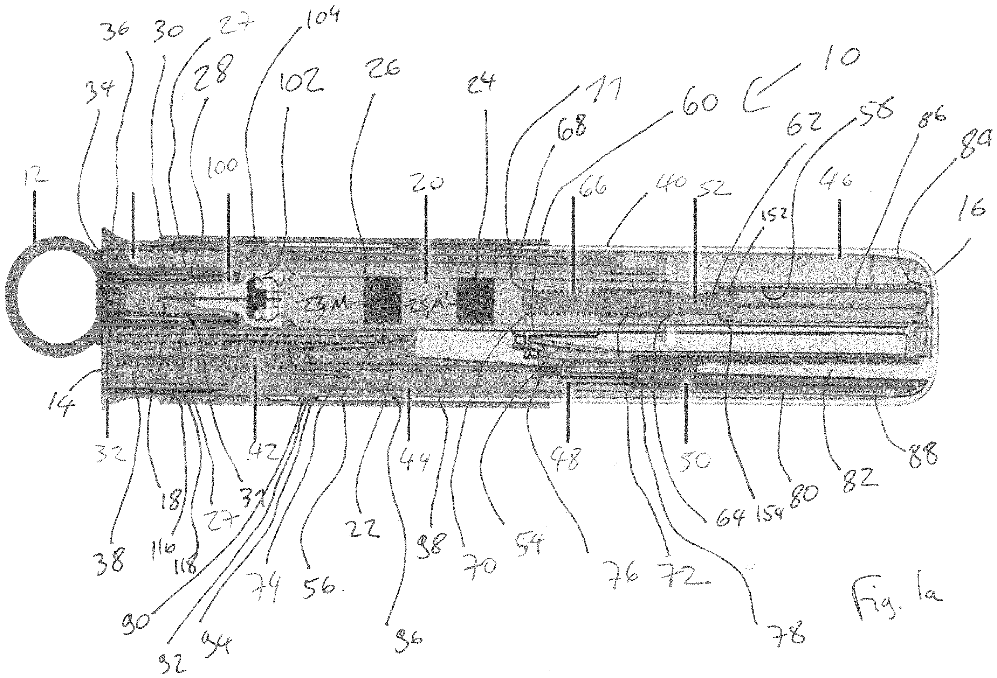

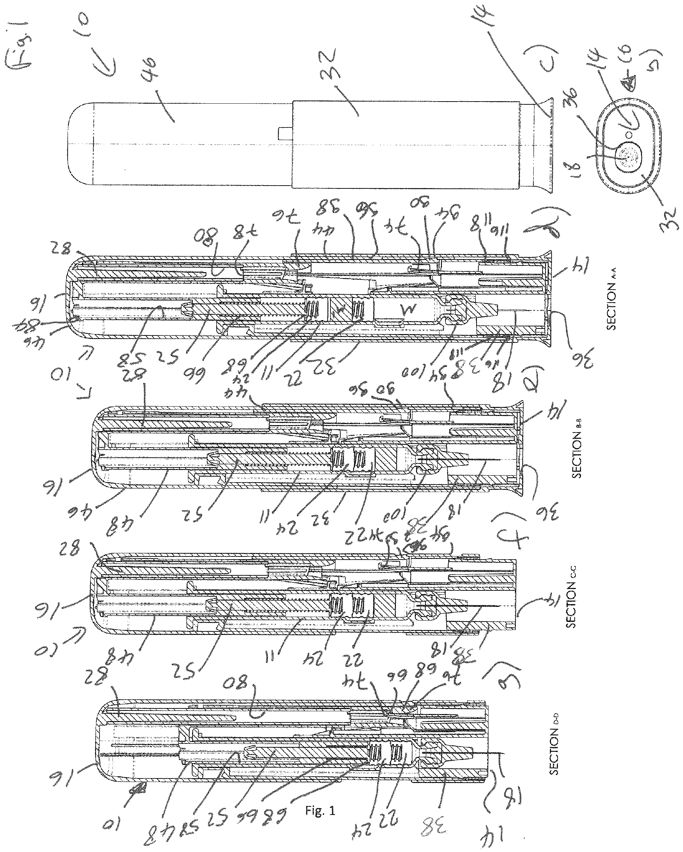

Fig. 1 shows various views of a dual chamber autoinjector 10. Fig. 1a shows the as delivered state of the autoinjector 10, with a handle 12 protruding from a proximal end 14 of the autoinjector 10. The proximal end 14 is oppositely disposed to the distal end 16.

In this connection it should be noted that the terms proximal and distal refer to the position of a cannula 18 of the autoinjector 10 relative to a patient (not shown) with proximal meaning closest to a main mass of the body of a patient and distal meaning it is more distant from the main mass of the body of a patient.

Hence, e.g. the proximal end 14 of the autoinjector 10 is that end that is placed at a patients skin with the distal end 16 being remote from the patient's skin in use, i.e. the end of the device having a needle respectively the cannula 18 that pierces a patient's skin is the proximal end 14.

The cannula 18 is arranged at a pre-filled syringe 20. The pre-filled syringe is a so-called dual chamber pre-filled syringe 20 having a dual chamber cartridge 11 which in a storage state comprises two chambers 23, 25 separated from one another for storage of two kinds of material M, M' by a proximal stopper 22, with the second chamber 25 also being bound by a distal stopper 24.

The autoinjector 10 further comprises a cap and seal assembly 100 at the dual chamber cartridge 11 covering the cannula 18.

Such a pre-filled syringe and hence the autoinjector 10 can be used for mixing, e.g. liquid/liquid compositions, respectively reconstitution processes, e.g. powder/liquid compositions, in which the two different materials M, M' are stored separate from one another but are mixed in a mixing or a reconstitution process to form a medicament before dispensing of the medicament. The reason for this is that the medicament may not have a long storage life once mixed but its components do.

The two chambers 23, 25 can be connected one to another via a bypass passage 26 once the distal stopper 24 and the proximal stopper are moved proximally towards the cannula 18.

The cannula 18 is covered by a cannula shield 28 that can be removed from the pre-filled syringe 20 on removal of the handle 12 from a cap 32 present at the proximal end 14 of the autoinjector 10.

The handle 12 comprises an inner cap 34 attached thereto, with the inner cap 34 being arranged within the cap 32. The inner cap 34 is configured to hold and move a metal pressing 30 arranged to hold and remove the cannula shield 28 on removal of the handle 12 from the cap 32.

In this connection it should be noted that the metal pressing 30 is formed by pressing a sheet metal part into an essentially U-shaped part with barbs 27 engaging the inner cap 34 and projections 31 engaging the cannula shield 28 for a removal thereof.

For this purpose the cap 32 comprises an aperture 36 via which the inner cap 34, the metal pressing 30 and the cannula shield 28 can be removed from the autoinjector 10 and the pre-filled syringe 20.

The autoinjector 10 further comprises a needle guard 38 that is configured to cover the needle, respectively the cannula 18 in different states of use. The needle guard 38 is configured to be moved relative to the cannula 18 once the cap 32 is removed (see e.g. difference in states of Figs. 1f and 1 g).

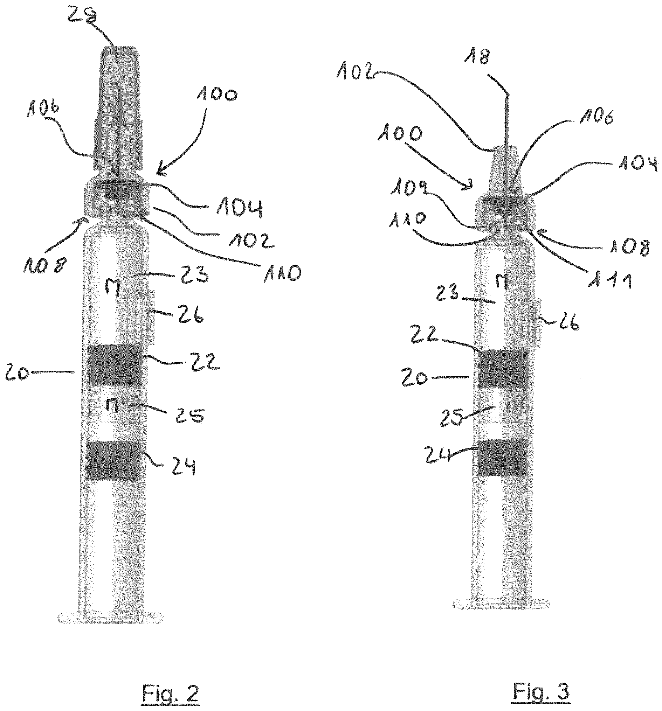

The needle guard 38 is arranged to move relative to a housing 40 of the autoinjector 10. A lock-out spring 42 is arranged between the needle guard 38 and the housing 40. In the state shown in Figs. 1 and 2a, the lock out 42 spring is in its extended the relaxed state whereas in the state indicated in Figs. 2b and 2c, the lock-out spring 42 is in the compressed state.

The housing 40 is formed by an inner body 44 and an outer body 46, with the pre-filled syringe being held at the inner body 44 which in turn is snap fit into place at the outer body 46 in such a manner that the pre-filled syringe cannot move axially with respect to either of the inner body 44 and the outer body 46 during a dispensing operation.

The autoinjector 10 further comprises a spring chassis 48 arranged movable in the housing 40 between a storage position (Figs. 1a, c, and d) respectively a pre-dispense position (Figs. 1e and 1f) and a dispensed position (see Fig. 1g).

A drive spring 50 is biased between the outer body 46 and the spring chassis 48 as a first biasing member. The first biasing member is configured to release energy in order to drive the spring chassis from the pre-dispense position to the dispensed position once the spring chassis 48 has been released for movement.

The drive spring 50 is arranged between the distal end 14 of the autoinjector 10 and a base 78 of a spring receiving space 80 of the spring chassis 48. The spring 50 is arranged about a spring projection 82 projecting proximally from an inner surface 84 of the outer body in the proximal direction. The inner surface 84 is present at the distal end 16 of the autoinjector 10.

The spring chassis 48 is released for movement relative to the housing 40 once the needle guard 38 is moved distally (see e.g. Fig. 1g) and a needle guard plunger 56 engages trigger arms 54 of the spring chassis 48 and releases an engagement between the trigger arms 54 and the housing 40.

In a storage state and in an activation state of the autoinjector 10, the spring chassis 48 is held with respect to the housing 10 via the trigger arms 54 that engage a corresponding structure 60 at the outer body 46.

The structure 60 can be a window 60 or a ledge 60 that holds the spring chassis 48 with respect to the outer body 46 against the bias of the drive spring 50.

In order to ensure an improved connection between the trigger arms 54 and the window 60 in the first and second configurations, the trigger arms 54 comprise projections 55 at their respective front ends. The projections 55 can also be used during a device lock-out in a third configuration, the lock-out state.

The spring chassis 48 then entrains a piston rod 52 in order to dispense a medicament composed of the mixed two kinds of material M, M'.

The inner body 44 comprises an end of dose click arm 66 that is configured to engage the spring chassis 48 towards an end of dose following the carrying out of the dispensing action. The end of dose click arm 66 cooperates with an end of dose click ramp 68 provided at the spring chassis 48. The end of dose click arm 66 can only engage the spring chassis once the spring chassis 48 has moved proximally.

The spring chassis 48 comprises a passage 62 relative to which the piston rod 52 can move. The passage 62 and the spring chassis 48 thereby act as a piston rod holder 58.

The piston rod 52 comprises a catch assembly 152 that can engage a distal end 154 of the passage 62.

The spring chassis 48 further comprises a ring seat 64 directly adjacent to the passage 62 in which a second biasing member 66 can sit and engage between the spring chassis 48 and a stopper 68 arranged at a front end 70 of the piston rod 52.

The second biasing member 66 sits in a ring channel 72 and surrounds the front end 70 of the piston rod 52 between the ring seat 64 and the stopper 68.

The spring chassis 48 is a U-shaped spring chassis 48 having first and second limbs 86, 88 extending in parallel to one another from the distal end 16 towards the proximal end 14 of the autoinjector 10 in use.

The first limb 86 is configured to hold and entrain the piston rod 52, whereas the second limb 88 is configured to hold and be entrained by the drive spring 50 once the spring chassis 48 is released from its engagement with the outer body 46.

The autoinjector 10 further comprises the cap 32 which can move relative to both the inner body 44 and the outer body 46 (see e.g. the difference in states of Fig. 1d and 1e) prior to removal of the cap from the autoinjector 10.

The inner body 44 comprises a snap fit projection 90 that extends transversely from the inner body 44. The snap fit projection 90 has a tapered surface 92 tapering outwardly from a more distal end towards the more proximal end of the snap fit projection 90.

The outer body 46 of the autoinjector 10 comprises first and second snap windows 94, 96 arranged next to one another, with the first snap window 94 being arranged closer to the proximal end 14 than the second snap window 96.

In the storage state of the autoinjector 10, the snap fit projection 90 engages the first snap window 94. Following a transfer of the autoinjector 10 from the storage state in a first configuration to a second activated state in a second configuration the inner body 44 and the outer body 46 are moved towards one another.

During this transfer from the first configuration to the second configuration, the cap 32 is moved towards the outer body 46 and the tapered surface 92 of the snap fit projection 90 ensures that the snap fit projection 90 allows disengagement of the snap fit projection 90 from the first snap window 94 so that the inner body 44 can be moved linearly towards the outer body 46 such that the snap fit projection can subsequently engage the second snap window 96.

The first and second snap windows 94, 96 are formed in an outer side surface 98 of the outer body 46 and are linearly spaced apart from one another in parallel to the pre-filled syringe 20.

In this connection it should be noted that the first and second snap windows 94, 96 are formed in the same outer side surface 98 of the outer body 46.

Fig. 1b shows a view of a bottom 98 of the cap 32 of the autoinjector 10 at the proximal end 14.

Fig. 1c shows a side view of the autoinjector 10 in the storage state. Figs. 1a and 1 d show corresponding sectional views of the autoinjector 10 of Fig. 1c.

A length of the cap 32 corresponds to approximately 40 to 70% of a length of the autoinjector 10.

The cap 32 is configured to hold the needle guard 28 relative to the inner body 44 during the storage state and the activation state. Only following removal of the cap 32 can the needle guard 28 be moved relative to the inner body 44.

Fig. 1d shows a state of the autoinjector 10 in which the inner cap 34 and the cannula shield 28 are removed prior to a reconstitution process having been carried out, i.e. a sectional view similar to that of Fig. 1a, with the inner cap 34 removed. The autoinjector 10 is still present in the first configuration.

Fig. 1e shows a state in which the cap 32 is moved distally relative to the housing 40 in order to move the distal plunger 24 and the second medicament M' proximally such that the second medicament M' can come into contact with the first medicament via the bypass passage 26 to carry out the reconstitution or mixing process, i.e. the autoinjector 10 is now in the second configuration where the snap fit projection 90 no longer engages the first snap window 94 but rather engages the second snap window 96.

In order to effect this transfer from the first configuration to the second configuration the cap 32 comprises two abutments 116 at a proximal end of the cap 32. The two abutments 116 are configured to engage a proximal end 118 of the inner body 44.

In use of the autoinjector 10 a user can for example place the distal end 16 of the autoinjector 10 against a surface, such as a table top, while holding the cap 32. Following placement at the surface, the user then pushes the cap 32 towards the surface in order to shift the inner body 44 towards the outer body and to reach the second configuration depicted in Fig. 1e.

Fig. 1f shows the state in which the cap 32 of the autoinjector 10 has been removed. Only after removal of the cap 32 is it possible to actuate the needle guard 38 which can be used to activate the autoinjector 10 for the dispensing process.

This occurs when the needle guard 38 is fully depressed and the needle guard plunger 56 engages the trigger arms 54 to release the spring chassis 48 during a dispensing operation.

As indicated in Figs. 1e and 1f, the proximal stopper and the distal stopper 22, 24 are close to or actually engage one another indicating that a reconstitution or mixing process has been completed and the autoinjector 10 is now activated for dispense.

As indicated in Fig. 1g, the needle guard 38 is fully depressed and the dispense is completed, i.e. the spring chassis 48 has moved as far proximally as possible.

During this proximal movement of the spring chassis 48 the, the end of dose click arm 74 engages the end of dose click ramp 76 and emits an audible clicking noise indicating a near completion respectively a completion of the dispensing process. The autoinjector 10 is now in the third configuration, the end of dispense state.

Once a patient hears the end of dose click, he knows that dispensing is completed and can remove the autoinjector 10 from the point of administration at the patient or start a count to do so, the lock-out spring 42 then moves the needle guard 38 into a locked position in which the cannula 18 is covered by the needle guard 38 (not shown). It is preferred if the needle guard 38 cannot be moved relative to the cannula 18 when this is in the locked position.

Figs. 2 and 3 show sectional views of a prefilled syringe 20 according to the invention. The syringe 20 of Fig. 2 additionally comprises the removable cannula shield 28 while the syringe 20 shown in Fig. 3 is without said cannula shield 28.

It can be clearly seen that the prefilled syringe 20 comprises a dual chamber cartridge 11 having two chambers 23, 25 wherein the first chamber 23 is arranged proximally relative to the second chamber 25. Each of said two chambers 23, 25 comprises one element M respectively M' of a medicament such that said two elements M, M' are stored separately from one another to enhance the storage time.

It can furthermore be clearly seen that the first and second chambers 23, 25 are separated from one another by the proximal stopper 22 and that the second chamber 25 additionally comprises a distal stopper 24 at its distal end.

The prefilled syringe 20 of Figs. 2 and 3 is shown in the storage state as the proximal stopper 22 is arranged behind, i.e. distally relative to the bypass passage 26 such that the material M' stored in the second chamber 25 cannot yet flow into the first chamber 23.

Additionally, the prefilled syringe 20 of Figs. 2 and 3 comprises the cap and seal assembly 100 having a cap 102 and one or more seals 104 with said cap and seal assembly 100 being fixed to and covering an opening 106 of the dual chamber cartridge 11.

It can be seen that the cap 102 completely surrounds seal 104 as well as the opening 106.

The cannula 18 extends through and beyond the cap and seal assembly 100 as it is depicted in Fig. 2. This way, the elements M, M' stored inside the dual chamber cartridge 11 can only flow through the cannula 18 during the act of dispensing as seal 104 completely seals and covers the potential open residue of the opening 106.

Said cannula 18 can furthermore be stabilized by cap 102 and/or by the one or more seals 104.

The cap and seal assembly 100 of Figs. 2 and 3 further comprises a first flange arrangement 108 while the dual chamber cartridge 11 comprises a second flange arrangement 110 that is shaped complementary to said first flange arrangement. The two flange arrangements 108,110 are engageable with one another such that the cap and seal assembly 100 can safely be secured to the dual chamber cartridge 11.

In the embodiment of Figs. 2 and 3 this is done by providing a first flange arrangement 108 with an undercut 109 and a second flange arrangement 110 with a web 111 such that said undercut 109 can engage with said web 111. In this case it is also possible that the first flange arrangement, i.e. the undercut 109, completely surrounds the second flange arrangement 110, i.e. the web 111.

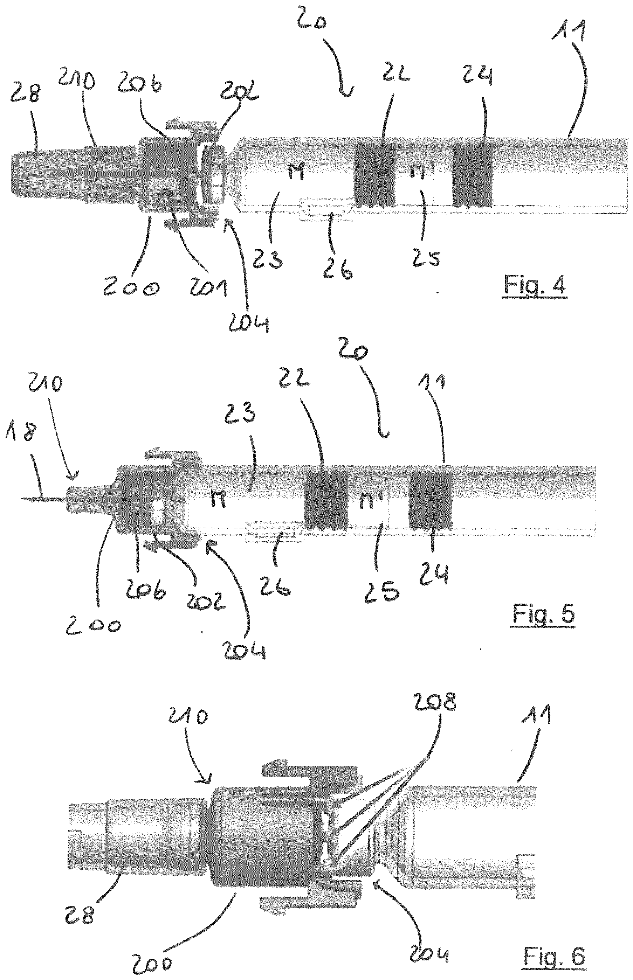

The second aspect of the invention is shown in Figs. 4 to 6 with Figs. 4 and 5 showing detailed sectional views of a prefilled syringe 20 according to this second aspect of the invention and Fig. 6 showing a detailed view of a cannula carriage 200 arranged at said prefilled syringe 20, in particular at the dual chamber cartridge 11.

In this connection it is noted that for the general features of the prefilled syringe 20 of Figs. 4 to 6 it is referred to the explanations made in connection with Figs. 1 to 4 as said general features are the same. In the following only the features that differ from the embodiments of Figs. 1 to 4 are described.

The prefilled syringe 20 according to Figs. 4 to 6 comprises a cannula carriage 200 instead of the cap and seal assembly 100 of Figs. 2 and 3. Said cannula carriage 200 is arranged at the outlet 106 of the dual chamber cartridge 11.

The cannula 18 is arranged inside of the cannula carriage such that it extends beyond said carriage 200 at a proximal end in the storage state of the autoinjector 10. At a distal end of the cannula carriage 200 said carriage 200 comprises a cannula carriage reception space 201 through which the cannula 18 extends. The cannula 18 is further arranged moveably with respect to said reception space 201 such that upon activation of the autoinjector 10 the dual chamber cartridge 11 is moved towards the cannula carriage 200.

Upon said activation movement the cannula 18 pierces a rubber septum 202 that is arranged at and seals the outlet 106 of the dual chamber cartridge 11 in the storage state. Hence, in the activated state of the autoinjector 10 the cannula carriage 200 is arranged closer to the dual chamber cartridge 11 such that the cannula 18 extends beyond the rubber septum 202 and thus the opening 106 of the dual chamber cartridge 11 into the first chamber 23.

At the distal end 204 of the carriage reception space 201 a seal 206 is arranged moveably with respect to said reception space 201 such that upon the activation process of the autoinjector 10 the seal 206 moves in a proximal direction towards a proximal end of the carriage reception space 201.

The seal 206 is arranged at the carriage reception space 201 to seal the area around the cannula 18. Furthermore, it may be configured to be pierced by the cannula 18 upon activation of the autoinjector 10 such that said seal 206 is pierced before the rubber septum 202 is pierced.

The cannula carriage 200 of Figs. 4 to 6 additionally comprises detents 208 at the distal end 204 thereof through which the cannula carriage 200 is in contact with the dual chamber cartridge 11 in the storage state of the autoinjector 11 (see in particular Fig. 6).

In the activated state of the autoinjector 10 the detents 208 can be overcome such that the cannula carriage 200 moves closer to the dual chamber cartridge 11 (see Fig. 5) to allow the piercing of the septum 202.

Also the prefilled syringe 20 according to Figs. 4 to 6 can comprise an additional cannula shield 28 that is arranged at the proximal end 210 of the cannula carriage 200 in the storage state of the autoinjector 10 (see Fig. 4). Upon activation of the autoinjector 10 the cannula shield 28 can be removed as it is already explained in connection with Fig. 1.

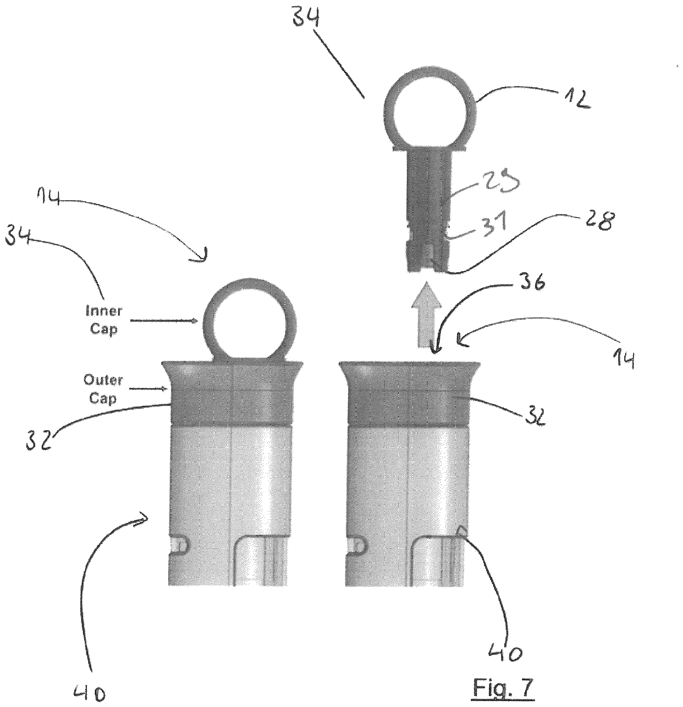

Another aspect of the invention is shown in Fig. 7 which shows a detailed view of the proximal end 14 of the autoinjector 10 comprising an inner and an outer cap 34, 32. The functioning of said inner and outer caps 34, 32 is already described in connection with Fig. 1.

In this connection it is noted that it may additionally be possible that the autoinjector according to Fig. 7, and thus also Fig. 1, additionally comprises the cannula carriage 200 as described in connection with Figs. 4 to 6 and/or the cap and seal assembly 100 as described in connection Figs. 2 and 3.

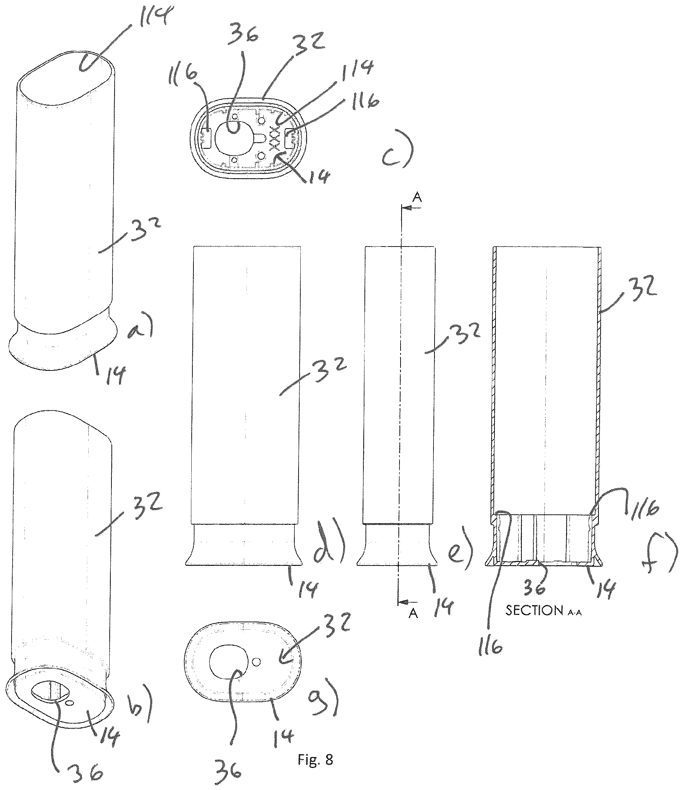

Fig. 8a to g show various views of the cap 32. A distal opening 114 of the cap 32 is visible. The autoinjector 10 is received within the cap 32 via the distal opening 114 on assembly of the autoinjector 10.

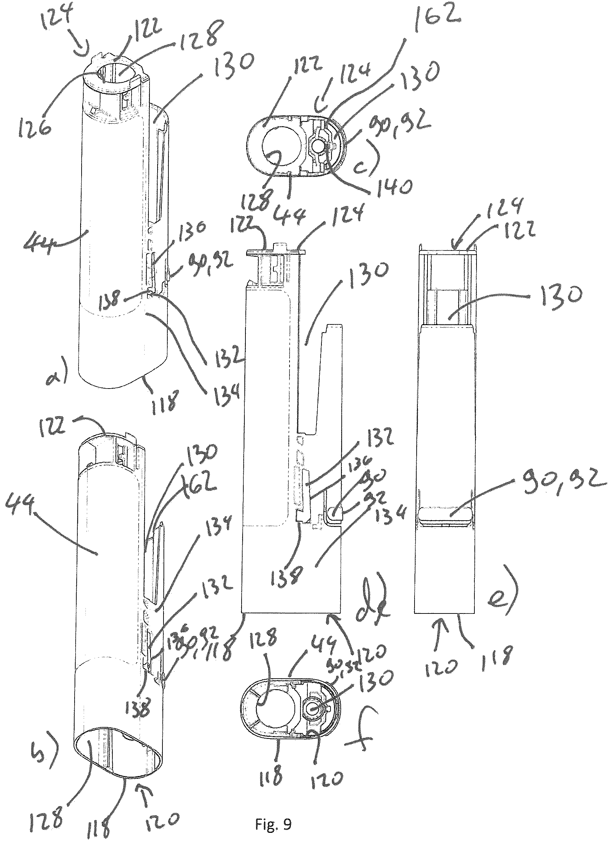

Figs. 9a to f show various views of the inner body 44. The proximal end 118 of the inner body 44 forms a proximal opening 120 through which and relative to which the needle guard 38 can move.

A distal end 124 of the inner body 44 comprises a syringe abutment 122 against which the pre-filled syringe 20 abuts. The syringe abutment 122 comprises an opening 126 through which the pre-filled syringe 20 passes into a syringe receiving space 128.

The distal end 124 of the inner body 44 may comprise deflection aids (not shown) in the region of the syringe abutment 122 that can counteract forces acting on the pre-filled syringe 20 when the spring chassis 48 is moved proximally relative to the pre-filled syringe 20.

The spring chassis 48 not only comprises the piston rod 52 at its first limb 86 that passes through the opening 126 during dispense, but also comprises the second limb 88 that likewise moves proximal. In order to accommodate the second limb 88, the inner body 44 also has a second receiving space 130 into which the second limb 88 comprising the trigger arms 54 are guided.

In order to ensure an end of dispense lock-out the inner body 44 comprises a lockout channel 132 in an outer wall 134 thereof. The lock out channel 132 comprises first and second part passages 136, 138 configured to accommodate the needle guard.

The first and second part passages 136, 138 are arranged inclined with respect to one another in such a way that the projection 55 is held in the second part passage following end of dispense as it is deflected from the first part passage 136 into the second part passage 138 via the trigger arms of the spring chassis.

A lock-out ramp 140 (see Fig. 9c) is also arranged within the inner body 44 at the proximal end 118 of the inner body 44 that acts as an end of dose click ramps.

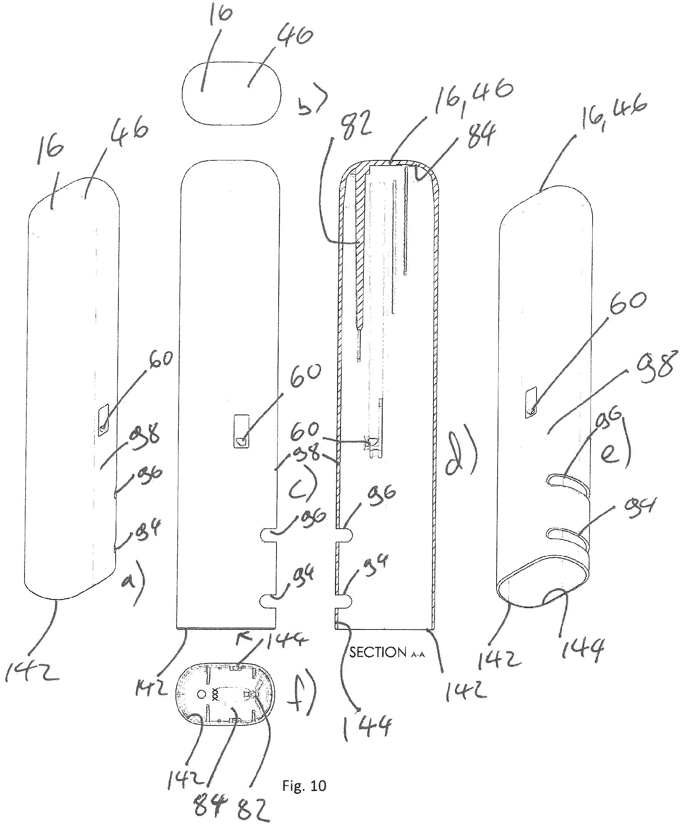

Figs. 10a to f show various views of the outer body 46. The outer body 46 extends between a proximal end 142 having a proximal opening 144 and the distal end 16 having the surface 84 at which e.g. the spring projection 82 is arranged.

On assembly of the autoinjector 10, the inner body 44 is moved into the outer body 46 with the pre-filled syringe 20, the spring chassis 48, the first and second biasing members 66 50 assembled and the inner body 44 is then snap fit into place between the snap fit projection 90 of the inner body 44 engaging the first snap window 94 of the outer body 46.

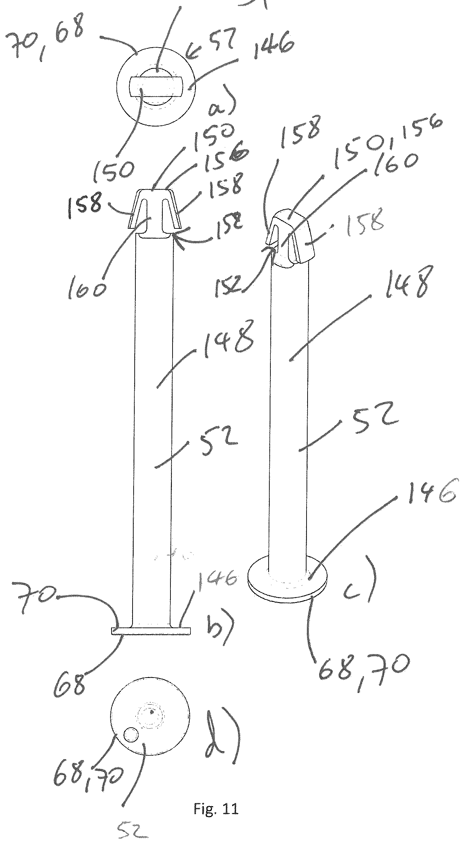

Figs. 11a to d show various views of the piston rod 52. The piston rod comprises the stopper 68 at its front end. A distal facing surface 146 of the piston rod 52 acts as a ring seat 146 for the second biasing member 66.

The piston rod 52 comprises a plunger section 148 extending between the rearward facing surface 146 of the stopper 68 and a rear end 150.

The catch assembly 152 is formed at the rear end 150 of the piston rod 52. The catch assembly 152 is configured to engage the rear end 154 of the passage 62 in order to ensure that the piston rod 52 cannot move proximally completely through the spring chassis 48 and become disengaged from the spring chassis 48 during an assembly of the autoinjector 10.

The catch assembly 152 comprises a hat 156 from which two arms 158 extend with the hat comprising a central limb 160 attached to the plunger section 148.

The arms 158 extend proximally away from the hat 156 at an inclination with respect to the central limb 160.

The central limb 160 has a reduced outer diameter with respect to the plunger section 148.

The reason for this is that the arms 158 can be urged through the passage 62 of the spring chassis 48 during an assembly of the autoinjector 10 and then can catch at the rear end 154 of the passage 62.

The first biasing member 66 is arranged about the plunger section 148 in an assembled state of the autoinjector 10.

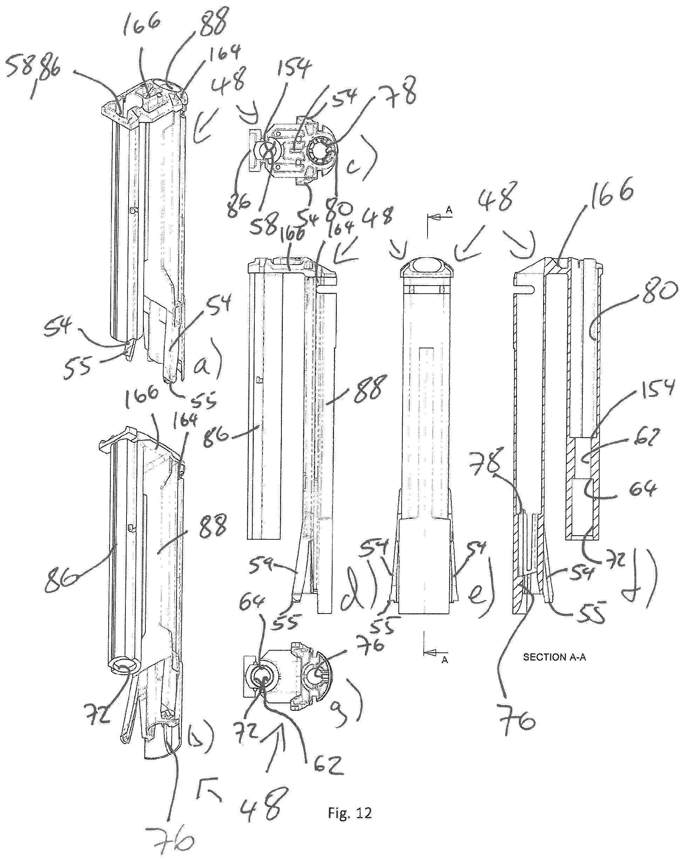

Figs. 12a to g show various views of the spring chassis 48.

A guide structure is formed between the spring chassis 48 and the inner body 44 in order to guide the spring chassis 48 with respect to the inner body 44. The guide structure comprises grooves 164 formed at the spring chassis 48 that engage complementary shaped splines 162 of the inner body 44 (sees Fig. 9b and 9c) for a linear guidance of the spring chassis 48 with respect to the inner body 44.

In this connection it should be noted that the inner body 44 could comprise grooves and the spring chassis 48 could comprise complementary shaped splines as a guiding structure.

The spring chassis 48 is a U-shaped spring chassis 48 having the first and second limbs 86, 88 extending in parallel to one another from the distal end 14 towards the proximal end 16 of the autoinjector 10 in use, with a bridge 166.

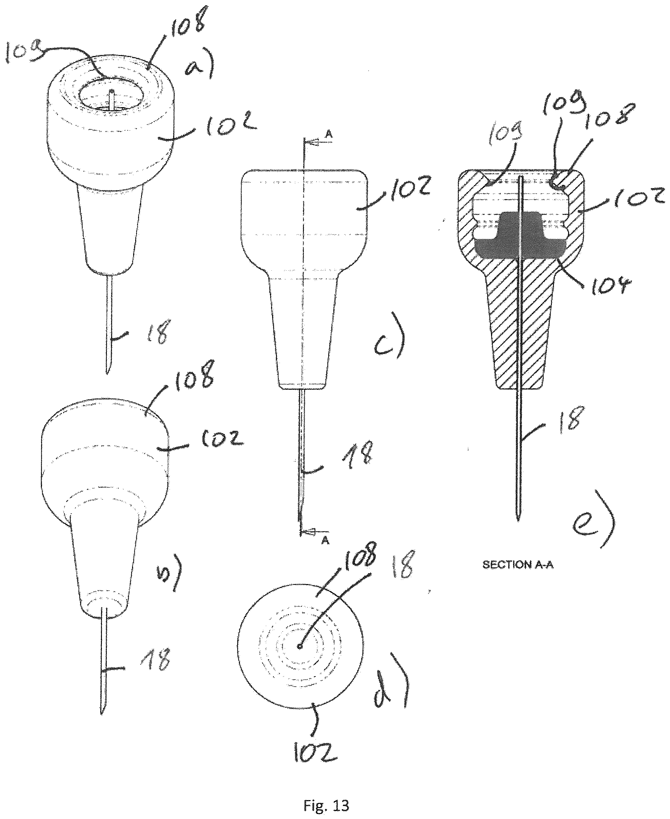

Fig. 13a to e show various views of a cap 102 of the cap and seal assembly 100 shown in Fig. 2 and 3. The cap 102 comprising the cannula 18 is configured to be attached to the opening 106 of the dual chamber cartridge 11. The seal 104 is provided in order to prevent medicament from escaping through the opening by means other than the cannula 18.

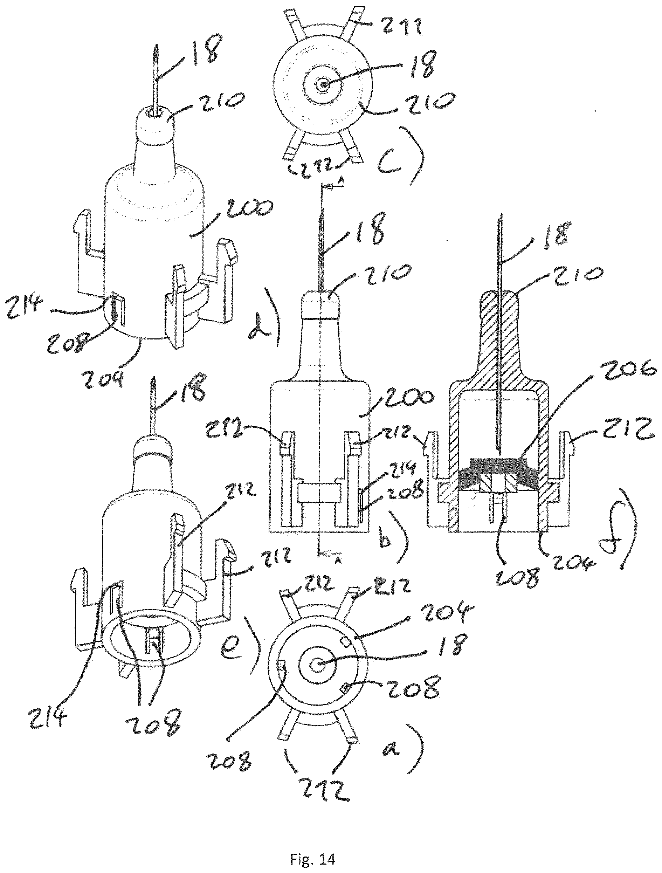

Fig. 14a to f show various views of a second kind of carriage 200. The difference to the carriage shown in Figs. 4 to 6 lies in the type of detents 208. Rather than projecting from the distal end 204, these are formed at the distal end 204 and are arranged in windows 214 of the carriage 200.

Also visible are wings 212 via which the carriage is snap fit into the inner body 44 of the autoinjector 10. The carriage is fixedly held in the autoinjector 10, so that on activating the autoinjector 10, the movement of the sleeve relative to the inner body 44, the dual chamber cartridge 11 is moved into engagement with the carriage 200 such that the cannula 18 pierces the seal 206 in order to facilitate the reconstitution process, i.e. the mixing process and to enable a dispensing from the dual chamber cartridge 11 after the mixing process has taken place.

On using the autoinjector 10 described in the foregoing, the user removes the inner cap 34 from the proximal end 14 of the autoinjector 10, places the distal end 16 of the outer body 46 onto a hard flat surface and presses down on the outer cap 32 until the inner body 44 clips fully into the outer body 46 and reaches a stop when the snap fit projection 90 engages the second snap window 94.

This movement between upper and lower sub-assemblies (i.e. outer body 46 and inner body 44) causes the assembly comprising the spring chassis 48 and the piston rod 52 to move proximally relative to the syringe 20. The piston rod 52 abuts the distal stopper 24, i.e. the rear end 152 of the passage 62, causing it to move proximally too (the pre-load in a piston rod spring 66 is sufficient to prevent the piston rod 52 from collapsing within the spring chassis 48) and forcing the contents of the distal chamber 25 through the bypass channel 26 and into the proximal chamber 23 to allow mixing of the various components of medicament M, M' stored in the pre-filled syringe 20.

The needle guard lockout mechanism of the autoinjector 10 comprising the trigger arms 54 and the lock-out channel 132, requires a certain travel of the spring chassis 48 during dispense (>26mm in the embodiment shown). This may be greater than the travel required to dispense the volume of medicament M, M' required.

The force from the extended piston rod spring 66 is higher than the force required to move the distal stopper 24 during mixing so that the assembly comprising the piston rod 52 and the spring chassis 48 is maintained at its full-length at least at the end of mixing, as necessary to move the distal stopper 24 as far as it needs to be moved.

The force from the compressed piston rod spring 66 is less than the force from the extended drive spring 50, so that even if the stopper travel required to dispense the dose is small, preventing further movement of the piston rod 52, the drive spring 50 is able to continue to move the spring chassis 48 (while compressing the piston rod spring 66) as far as needed to engage the needle guard lockout mechanism.

This over travel allows the spring chassis 48 to complete its minimum required displacement, even for variants with small volumes of dispensed medicament.

### List of reference numerals:

- 10: autoinjector
- 11: dual chamber cartridge
- 12: handle
- 14: proximal end
- 16: distal end
- 18: cannula
- 20: pre-filled syringe
- 22: proximal stopper
- 23: first chamber
- 24: distal stopper
- 25: second chamber
- 26: bypass passage
- 27: barb
- 28: removable cannula shield
- 29: cannula shield holder
- 30: metal pressing
- 31: projections
- 32: cap
- 34: inner cap
- 36: aperture
- 38: needle guard
- 40: housing
- 42: lock-out spring
- 44: inner body
- 46: outer body
- 48: spring chassis
- 50: drive spring/first biasing member
- 52: piston rod
- 54: trigger arms
- 55: trigger arm projection
- 56: needle guard plunger
- 58: piston rod holder
- 60: structure/window
- 62: passage
- 64: ring seat
- 66: second biasing member
- 68: stopper
- 70: front end of 52
- 72: ring channel
- 74: end of dose click arm
- 76: end of dose click ramp
- 78: base of 80 in 48
- 80: spring receiving space
- 82: spring projection
- 84: inner end surface of 46
- 86: first limb of 48
- 88: second limb of 48
- 90: snap fit projection
- 92: tapered surface of 90
- 94: first snap window
- 96: second snap window
- 98: side surface of 46
- 100: cap and seal assembly
- 102: cap
- 104: seal
- 106: opening
- 108: first flange arrangement
- 109: undercut
- 110: second flange arrangement
- 111: web
- 112: rubber septum
- 114: opening of 32
- 116: abutment
- 118: proximal end of 44
- 120: proximal opening of 44
- 122: syringe abutment
- 124: distal end of 44
- 126: opening
- 128: syringe receiving space
- 130: second receiving space
- 132: lock-out channel
- 134: outer wall
- 136: first part passage
- 138: second part passage
- 140: lock-out ramp
- 142: proximal end of 46
- 144: proximal opening
- 146: rearward facing surface of 68
- 148: plunger section
- 150: rear end of 52
- 152: catch assembly
- 154: rear end of 62
- 156: hat
- 158: arms
- 160: central limb
- 162: groove
- 164: spline
- 166: bridge
- 200: cannula carriage
- 201: cannula carriage reception space
- 202: rubber septum
- 204: distal end
- 206: seal
- 208: detent
- 210: proximal end
- 212: wings
- 214: window

### Enumerated embodiments:

1. An autoinjector comprising a dual chamber component comprising first and second chambers for storing first and second elements of a medicament that can each be stored in the respective first and second chambers of the dual chamber component, a piston rod, an inner body and an outer body, wherein the inner body and the outer body are in engagement with one another in a first configuration in a storage state and, on activation of the autoinjector are moved towards one another and then fixedly clipped to one another in a second configuration.
2. The autoinjector of embodiment 1, wherein the inner body and the outer body are clipped to one another in the first configuration.
3. The autoinjector of embodiment 1, wherein the inner body and the outer body are resting with respect to one another in the first configuration.
4. The autoinjector of one of embodiments 1 to 3, wherein on moving the inner body towards the outer body, the piston rod is moved into the dual chamber component for a mixing of medicament on the transition from the first configuration into the second configuration.
5. The autoinjector of one of embodiments 1 to 4, wherein, on dispensing a medicament, the autoinjector is transferred from the second configuration into a third configuration.
6. The autoinjector of one of embodiments 1 to 5, further comprising a needle guard moveable relative to the dual chamber component.
7. The autoinjector of one of embodiments 1 to 6, further comprising a spring chassis, with the spring chassis being configured to be non-moveable held in the first configuration in at least one of the inner body and the outer body and with the spring chassis being configured to be moved relative to both the inner body and the outer body once the autoinjector is transferred from the second configuration into a third configuration.
8. The autoinjector of embodiment 6 and embodiment 7, wherein a proximal movement of said spring chassis is initiated by a distal movement of said needle guard.
9. The autoinjector of one of embodiments 7 to 8, wherein the spring chassis is moved in the autoinjector for a dispensing operation of the autoinjector.
10. The autoinjector of one of embodiments 7 to 9, wherein the piston rod is biased with respect to the spring chassis by a first biasing member.
11. The autoinjector of one of embodiments 7 to 10, wherein the spring chassis is biased with respect to the outer body by a second biasing member.
12. The autoinjector of embodiment 11, wherein the spring chassis is biased within the autoinjector during the first and second configurations with respect to said second biasing member.
13. The autoinjector of embodiment 12, wherein the spring chassis is fixed within the autoinjector with respect to the spring bias of the second biasing member in the second configuration.
14. The autoinjector of one of embodiments 9 to 13, wherein a force of the second biasing member is greater than a force of the first biasing member.
15. The autoinjector of one of embodiments 9 to 14, wherein the first biasing member is a first compression spring.
16. The autoinjector of embodiment 15, wherein the first compression spring has a spring force selected in the range of 3 to 5 N.
17. The autoinjector of one of embodiments 11 to 16, wherein the second biasing member is a second compression spring.
18. The autoinjector of embodiment 18, wherein the second compression spring has a spring force selected in the range of 5 to 40 N.
19. The autoinjector of one of embodiments 7 to 18, wherein the piston rod is arranged within the spring chassis and moveable relative thereto as well as being entrainable by the spring chassis.
20. The autoinjector of embodiment 19, wherein the second biasing member is arranged partly about the piston rod and at least partly within the spring chassis.
21. The autoinjector of one of embodiments 7 to 20 and embodiment 10, wherein, when the spring chassis is initially moved relative to the inner body and the outer body in the second configuration, the spring chassis is moved relative to the piston rod until the spring chassis has compressed the first biasing member in order to entrain the piston rod together with the spring chassis for a dispensing operation.
22. The autoinjector of one of embodiments 1 to 21, further comprising a cap assembly having an outer cap.
23. The autoinjector of embodiment 22, wherein a dispensing operation of the autoinjector can only take place once the outer cap is removed.
24. The autoinjector of embodiment 22 or embodiment 23, wherein the cap assembly further has an inner cap.
25. The autoinjector of embodiment 24, wherein the autoinjector can only be moved from the first configuration into the second configuration after removal of the inner cap from the autoinjector.
26. The autoinjector of embodiment 24 or embodiment 25, wherein the dual chamber component comprises a cannula covered by a needle shield and the inner cap is configured to hold the needle shield.
27. The autoinjector of embodiment 26, wherein the needle shield is removed from the autoinjector on removal of the inner cap.
28. The autoinjector of one of embodiments 14 to 27, wherein the first biasing member can be compressed during a dispensing operation, with a compression length of the first biasing member corresponding to a distance of travel of the spring chassis with respect to the piston rod on transferring the autoinjector from the second configuration to the third configuration.
29. The autoinjector of embodiment 28, wherein the compression length can be selected in a range of 10 to 40 mm.
30. The autoinjector of embodiment 28 or embodiment 29, wherein once the spring chassis has travelled the compression length with respect to the piston rod, the spring chassis is configured to entrain the piston rod.
31. The autoinjector of one of embodiments 5 and 6 to 30, wherein a distance of travel of the spring chassis on a transfer of the autoinjector from the second configuration into the third configuration is longer than a distance of travel of the piston rod.
32. The autoinjector of embodiment 31 and one of embodiments 28 to 30, wherein the longer distance of travel of the spring chassis with respect to the piston rod at least essentially corresponds to the compression length or corresponds to the compression length.
33. An autoinjector, in particular in accordance with one of the preceding embodiments, the autoinjector comprising a housing, a spring chassis and a piston rod, wherein the piston rod is moveably arranged within the spring chassis and biased with respect to the spring chassis by means of a first biasing member, wherein the spring chassis is biased with respect to the housing by means of a second biasing member, and wherein a force of the second biasing member is greater than a force of the first biasing member.
34. The autoinjector of embodiment 33, wherein, when the spring chassis is initially moved relative to the inner body and the outer body in the second configuration, the spring chassis is moved relative to the piston rod until the spring chassis has compressed the first biasing member in order to entrain the piston rod together with the spring chassis for a dispensing operation.
35. The autoinjector of embodiment 33 or embodiment 34, wherein the first biasing member can be compressed during the dispensing operation, with a compression length of the first biasing member corresponding to a distance of travel of the spring chassis with respect to the piston rod on transferring the autoinjector from the second configuration to the third configuration.
36. The autoinjector of embodiment 35, wherein the compression length can be selected in a range of 10 to 40 mm.
37. The autoinjector of embodiment 28 or embodiment 29, wherein once the spring chassis has travelled the compression length with respect to the piston rod, the spring chassis is configured to entrain the piston rod.
38. The autoinjector of one of embodiments 33 to 37, wherein a distance of travel of the spring chassis on a transfer of the autoinjector from the second configuration into the third configuration is longer than a distance of travel of the piston rod.
39. The autoinjector of one of embodiments 35 to 38, wherein the longer distance of travel of the spring chassis with respect to the piston rod at least essentially corresponds to the compression length or corresponds to the compression length.
40. A method of activating an autoinjector, in particular an autoinjector in accordance with one of the preceding embodiments, the autoinjector comprising an inner cap, an outer cap, an inner body, and an outer body, the method comprising the steps of: removing said inner cap from a proximal end of said autoinjector;
   pressing the outer cap to move the inner body relative to the outer body; and
   fixing said inner body to said outer body.
41. An autoinjector comprising a dual chamber cartridge (11) comprising two elements of a medicament (M, M') that are each stored in a separate chamber (23, 24) of the dual chamber cartridge (11) and the dual chamber cartridge (11) comprising a bypass passage (26) and the autoinjector (10) further comprising at least one security mechanism configured to secure the autoinjector against premature mixing of the elements and dispensing of said mixed elements (M, M').
42. An autoinjector, in particular according to embodiment 41, comprising a dual chamber cartridge (11) comprising two elements of a medicament (M, M') that are each stored in a separate chamber (23, 24) of the dual chamber cartridge (11) and the dual chamber cartridge (11) comprising a bypass passage (26), the dual chamber cartridge (11) comprising a syringe cap and seal assembly (100) covering and fixed to an outlet from the dual chamber cartridge (11).
43. The autoinjector of embodiment 42, wherein the cap and seal assembly (100) comprises a cap (102) and one or more seals (104) surrounded by the cap (102).
44. The autoinjector of embodiment 43, wherein the dual chamber cartridge (11) has an opening (106) which is covered by the cap (102) and sealed off by one of said one or more seals (104) of the cap and seal assembly (100).
45. The autoinjector of one of embodiments 42 to 44, wherein the cap and seal assembly (100) comprises a first flange arrangement (108) and the dual chamber cartridge (11) comprises a second flange arrangement (110) shaped complementary to the first flange arrangement (108) and being engageable by the first flange arrangement (108).
46. The autoinjector of embodiment 45, wherein the first flange arrangement (108) surrounds said second flange arrangement (110).
47. The autoinjector of embodiment 45 or embodiment 46, wherein the first flange arrangement (108) is snap-fit into place at the second flange arrangement (110).
48. The autoinjector of one of embodiments 42 to 47, further comprising a cannula (18) held in place at the dual chamber cartridge (11) by the cap and seal assembly (100).
49. The autoinjector of embodiment 8 and one of embodiments 43 to 47, wherein the cannula (18) is held in place at the dual chamber cartridge (11) by one of said one or more seals (104) of the cap and seal assembly (100).
50. The autoinjector of one of embodiments 42 to 49, wherein the cap and seal assembly (100) further comprises a cannula shield (28) covering the cap and seal assembly (100).
51. The autoinjector of one of embodiments 42 to 50, wherein the dual chamber cartridge (11) further comprises a proximal (22) and a distal stopper (24).
52. The autoinjector of one of embodiments 42 to 51, wherein the dual chamber cartridge (11) comprises a first chamber (23) more proximally arranged than a second chamber (25), wherein the first chamber (23) is filled with a solid material (M) and the second chamber (25) is filled with a liquid material (M').
53. The autoinjector of embodiments 51 and embodiment 52, wherein the first chamber (23) is separated from the second chamber (25) by the proximal stopper (22).
54. The autoinjector of one of embodiments 51 to 53, wherein the proximal stopper (22) is arranged distally of the bypass passage (26) in a storage state of the autoinjector (10).
55. The autoinjector of one of embodiments 51 to 54, wherein the proximal stopper (22) is arranged proximally of the bypass passage (26) after a dispensing operation has taken place.
56. The autoinjector of one of embodiments 42 to 55, further comprising a piston rod (52) configured to act on said distal stopper (24) and said proximal stopper (22) and to thereby move the proximal stopper (22) such that it is arranged at the bypass passage (26) for urging, i.e. allowing the material into the proximal chamber (23) past the proximal stopper (22) via the bypass passage (26) during a mixing process.
57. The autoinjector of embodiment 56, wherein the piston rod (52) can be moved further for dispensing the medicament via the cap and seal assembly (100).
58. The autoinjector of one of embodiments 44 to 57, wherein the one of said one or more seals (104) of the cap and seal assembly (100) sealing off the opening (106) covers said opening (106).
59. The autoinjector of one of embodiments 42 to 58, further comprising a cannula shield (28) such as a rigid cannula shield (28) covering a cannula (18) of the pre-filled syringe.
60. A method of mixing a medicament in an autoinjector (10), the autoinjector (10) comprising a dual chamber cartridge (11) comprising first and second elements (M, M') of a medicament, with the first and second elements (M, M') each being stored in a separate chamber (23, 25) of the dual chamber cartridge (11), the dual chamber cartridge (11) comprising a cap and seal assembly (100) as well as a piston rod (52), the method comprising the steps of:
   - moving the piston rod (52) proximally towards a distal stopper (24) and thereby moving said distal stopper (24) towards a proximal stopper (22) and reducing the distance between the distal stopper (24) and said proximal stopper (22) to thereby urge a first element (M) of the medicament from a first chamber (23) into a bypass passage (26) to bypass the proximal stopper (25) such that the first element (M) comes into contact with the second element (M') in the first chamber (25) arranged between the cap and seal assembly (100) and the proximal stopper (22).
61. An autoinjector, in particular according to embodiment 41, comprising a dual chamber cartridge (11) comprising two elements (M, M') of a medicament that are each stored in a separate chamber (23, 25) of the dual chamber cartridge (11), the dual chamber cartridge (11) comprising a bypass passage (26) and a rubber septum (202) and a cannula shield (28), the autoinjector (10) further comprising a cannula carriage (200) having a cannula (18) with the cannula carriage (200) being in contact with the dual chamber cartridge (11) in a storage state of the autoinjector (10) and the dual chamber cartridge (11) being moveable towards the cannula carriage (200) on activation of the autoinjector (10) in order to penetrate the septum (202) with the cannula (18).
62. The autoinjector of embodiment 61, wherein a distal end (204) of the cannula carriage (200) is sealed off by a seal (206).
63. The autoinjector of embodiment 62, wherein the seal (206) is moveably arranged in a distal cannula carriage reception space (201) relative to the cannula (18).
64. The autoinjector of one of embodiments 61 to 63, wherein the cannula carriage (200) comprises one or more detents (208) at a distal end (204) thereof.
65. The autoinjector of embodiment 64, wherein the cannula carriage (200) is in contact with the dual chamber cartridge (11) via the one or more detents (208) in the storage state of the autoinjector (10).
66. The autoinjector of embodiment 64 or embodiment 65, wherein, on activation of the autoinjector (10), the dual chamber cartridge (11) is configured to overcome said one or more detents (208) such that the cannula (18) pierces the septum (202).
67. The autoinjector of one of embodiments 62 to 66, wherein a movement of the dual chamber cartridge (11) towards the cannula carriage (200) moves said seal (206) towards the cannula (18) to pierce said seal (206) on activation of the autoinjector (10).
68. The autoinjector of embodiment 64 and one of embodiments 62 to 66, wherein the movement of the dual chamber cartridge (11) towards the cannula carriage (200) moves said seal (206) towards the cannula (18) to first pierce said seal (206) and then secondly pierce said septum (202).
69. The autoinjector of one of embodiments 61 to 68, further comprising a distal stopper (24) arranged in said dual chamber cartridge (11), and a piston rod (52), wherein, on activation of said autoinjector (10), a movement of said piston rod (52) towards the distal stopper (24) brings about a movement of said dual chamber cartridge (11) towards said cannula carriage (200).
70. The autoinjector of one of embodiments 64 to 67 and embodiment 69, wherein, once said septum (202) is pierced, movement of said distal stopper (24) within the dual chamber cartridge (11) is facilitated.
71. The autoinjector of one of embodiments 61 to 69, wherein the dual chamber cartridge (11) comprises a proximal (22) and a distal stopper (24) and a bypass passage (26).
72. The autoinjector of one of embodiments 61 to 70, wherein the dual chamber cartridge (11) comprises a first chamber (23) more proximally arranged than a second chamber (25), wherein the first chamber (23) is filled with a solid material (M) of said medicament and the second chamber (25) is filled with a liquid material (M') of said medicament.
73. The autoinjector of embodiments 71 and embodiment 72, wherein the first chamber (23) is separated from the second chamber (25) by the proximal stopper (22).
74. The autoinjector of one of embodiments 71 to 73, further comprising a piston rod (52) configured to act on said distal stopper (24) and said proximal stopper (22) and to thereby move the proximal stopper (22) such that it is arranged at the bypass passage (26) for urging the liquid material (M') into the first chamber (23) past the proximal stopper (22) via the bypass passage (26).
75. A method of activating an autoinjector, the autoinjector (10) comprising a dual chamber cartridge (11) comprising first and second elements (M, M') of a medicament, with the first and second elements (M, M') each being stored in a separate chamber (23, 25) of the dual chamber cartridge (11), a cannula carriage (200) having a cannula (18) and a piston rod (52), the method comprising the steps of:
   - moving the piston rod (52) proximally towards a distal stopper (24) arranged in the dual chamber cartridge (11) and thereby moving said dual chamber cartridge (11) towards the cannula carriage (200) to pierce a septum (202) of said dual chamber cartridge (11) with said cannula (18).
76. The method of embodiment 75, further comprising the step of:
   allowing air to be expelled from said dual chamber cartridge (11) via said cannula (18) and then further moving the distal stopper (24) towards a proximal stopper (22) to thereby urge a second element (M') of the medicament from a second chamber (25) into a bypass passage (26) to bypass the proximal stopper (22) such that the second element (M') comes into contact with the first element (M) in the first chamber (23) arranged between the cannula carriage (200) and the proximal stopper (22).
77. An autoinjector, in particular according to embodiment 41, comprising a dual chamber cartridge (11) comprising two elements (M, M') of a medicament that are each stored in a separate chamber (2, 25) of the dual chamber cartridge (11) and the dual chamber cartridge (11) comprising a bypass passage (26) and an outer cap (34), an inner cap (34) and a cannula shield (28) covering a cannula (18) of the autoinjector (10), wherein, on activation of the autoinjector (10) said inner cap (34) is removable from the autoinjector (10) before said outer cap (34) is removed from said autoinjector (10).
78. The autoinjector of embodiment 77, wherein the autoinjector (10) is configured such that removal of the inner cap (34) simultaneously brings about a removal of the cannula shield (28).
79. The autoinjector of embodiment 77 or embodiment 78, wherein the inner cap (34) comprises a grip (12) projecting from the autoinjector (10).
80. The autoinjector of embodiment 79 wherein the grip (12) comprises a ring.
81. The autoinjector of one of embodiments 77 to 80, wherein removal of the inner cap (34) permits a movement of air out of the dual chamber cartridge (11).
82. The autoinjector of one of embodiments 77 to 81, wherein the inner cap (34) comprises a cannula shield holder (29) at an end of the inner cap (34) disposed opposite to a base of the inner cap (34).
83. The autoinjector of one of embodiments 77 to 82, wherein the inner cap (34) comprises inwardly facing projections (31) that engage the cannula shield (28).
84. The autoinjector of embodiment 82 and embodiment 83, wherein the inwardly facing projections (31) are integrally formed with the cannula shield holder (29) of the inner cap (34).
85. The autoinjector of embodiment 82 and embodiment 83, wherein the inwardly facing projections are formed by a metal insert (30) received within the cannula shield holder (29) of the inner cap (34).
86. The autoinjector of one of embodiments 77 to 85, wherein the outer cap (34) is configured to receive a part of a cannula shield (28) of the autoinjector (10).
87. The autoinjector of one of embodiments 77 to 86, wherein an axial movement of a needle guard (38) of the autoinjector (10) in the direction of the dual chamber cartridge (11) brings about a release of a drive mechanism of a plunger of the dual chamber cartridge (11) for dispensing a medicament (M, M') stored in the dual chamber cartridge (11).
88. The autoinjector (10) according to one of the preceding embodiments, wherein the outer body comprises first and second snap fit windows (94, 96) and the inner body (44) comprises a snap fit projection (90), wherein the snap fit projection (90) engages the first snap window (94) in the first configuration and the snap fit projection (90) engages the second snap window (96) in the second configuration.
89. The autoinjector (10) according to embodiment 88, wherein the first and second snap fit windows (94, 96) are linearly spaced apart from one another in an outer side surface (98) of the outer body (46).
90. The autoinjector (10) according to embodiment 88 or embodiment 89, wherein the snap fit projection (90) comprises a tapered surface tapering outwardly form a distal end (16) to a proximal end (14) of the autoinjector (10).
91. The autoinjector (10) according to one of embodiments 1 to 90, further comprising a catch assembly (152) at a rear end of the piston rod (52).
92. The autoinjector (10) according to embodiment 91, wherein the catch assembly comprises at least two arms (158) inclined with respect to the main extent of the piston rod (52).

## Claims

1. An autoinjector (10) comprising a dual chamber component (11) comprising first and second chambers (23, 25) for storing first and second elements of a medicament (M, M') that can each be stored in the respective first and second chambers (23, 25) of the dual chamber component (11), a piston rod (52), an inner body (44) and an outer body (46), wherein the inner body (44) and the outer body (46) are in engagement with one another in a first configuration in a storage state and, on activation of the autoinjector (10) are moved towards one another and then fixedly clipped to one another in a second configuration.

2. The autoinjector (10) of claim 1, wherein the inner body (44) and the outer body (46) are clipped to one another in the first configuration; and/or wherein the inner body (44) and the outer body (46) are resting with respect to one another in the first configuration.

3. The autoinjector (10) of claim 1 or claim 2, wherein on moving the inner body (44) towards the outer body (46), the piston rod (52) is moved into the dual chamber component (11) for a mixing of medicament on the transition from the first configuration into the second configuration.

4. The autoinjector (10) of one of claims 1 to 3, wherein, on dispensing a medicament, the autoinjector (10) is transferred from the second configuration into a third configuration.

5. The autoinjector (10) of claim 4, further comprising a spring chassis (48), with the spring chassis (48) being configured to be non-moveable held in the first configuration in at least one of the inner body (44) and the outer body (46) and with the spring chassis (48) being configured to be moved proximally relative to both the inner body (44) and the outer body (46) once the autoinjector is transferred from the second configuration into the third configuration.

6. The autoinjector (10) of claim 4 or claim 5, further comprising a needle guard (38), wherein a proximal movement of said spring chassis (48) is initiated by a distal movement of the needle guard (38).

7. The autoinjector (10) of claim 5 or claim 6, wherein the piston rod (52) is biased with respect to the spring chassis (48) by a first biasing member (66).

8. The autoinjector (10) of one of claims 5 to 7, wherein the spring chassis (48) is biased with respect to the outer body (46) by a second biasing member (50), in particular wherein the spring chassis (48) is biased within the autoinjector (10) during the first and second configurations with respect to said second biasing member (50), especially wherein the spring chassis (48) is fixed within the autoinjector (10) with respect to the spring bias of the second biasing member (50) in the second configuration.

9. The autoinjector (10) of claim 7 and claim 8, wherein a force of the second biasing member (50) is greater than a force of the first biasing member (66).

10. The autoinjector (10) of one of claims 7 to 9, wherein the first biasing member (66) is a first compression spring (66), in particular wherein the first compression spring (66) has a spring force selected in the range of 3 to 5 N.

11. The autoinjector (10) of one of claims 8 to 10, wherein the second biasing member (50) is a second compression spring (50), in particular wherein the second compression spring (50) has a spring force selected in the range of 5 to 40 N.

12. The autoinjector (10) of one of claims 5 to 11, wherein the piston rod (52) is arranged within the spring chassis (48) and moveable relative thereto as well as being entrainable by the spring chassis (48); and/or
wherein the first biasing member (66) is arranged partly about the piston rod (52) and at least partly within the spring chassis (48); or
wherein the piston rod is configured as a tube outside of a shaft of the spring chassis, with the first biasing member being arranged within the tube and about the shaft of the spring chassis.

13. The autoinjector (10) of one of claims 5 to 12, wherein, when the spring chassis (48) is initially moved relative to the inner body (44) and the outer body (46) on transferring the autoinjector (10) from the second configuration into the third configuration, the spring chassis (48) is moved relative to the piston rod (52) until the spring chassis (48) has compressed the first biasing member (66) in order to entrain the piston rod (52) together with the spring chassis (48) for a dispensing operation.

14. The autoinjector (10) of one of claims 1 to 21, further comprising a cap assembly having an outer cap (32) and wherein a dispensing operation, i.e. when the autoinjector (10) is transferred from the second configuration to the third configuration, of the autoinjector (10) can only take place once the outer cap (32) is removed, optionally wherein the cap assembly further has an inner cap (34) and wherein the autoinjector (10) can only be moved from the first configuration into the second configuration after removal of the inner cap (34) from the autoinjector (10).

15. The autoinjector (10) of one of claims 7 to 14, wherein the first biasing member (66) can be compressed during a dispensing operation, with a compression length of the first biasing member (66) corresponding to a distance of travel of the spring chassis (48) with respect to the piston rod (52) on transferring the autoinjector (10) from the second configuration to the third configuration, in particular wherein the compression length can be selected in a range of 0 to 40 mm, especially wherein, once the spring chassis (48) has travelled the compression length with respect to the piston rod (52), the spring chassis (48) is configured to entrain the piston rod (52).

16. The autoinjector (10) according to one of claims 1 to 15, wherein the outer body (46) comprises first and second snap fit windows (94, 96) and the inner body (44) comprises a snap fit projection (90), wherein the snap fit projection (90) engages the first snap window (94) in the first configuration and the snap fit projection (90) engages the second snap window (96) in the second configuration.

17. The autoinjector (10) according to claim 16, wherein the first and second snap fit windows (94, 96) are linearly spaced apart from one another in an outer side surface (98) of the outer body (46)

18. An autoinjector (10), in particular in accordance with one of the preceding claims, the autoinjector comprises a housing, a spring chassis (48) and a piston rod (52), wherein the piston rod (52) is moveably arranged within the spring chassis (48) and biased with respect to the spring chassis (48) by means of a first biasing member (66), wherein the spring chassis (48) is biased with respect to the housing by means of a second biasing member (50), and wherein a force of the second biasing member (50) is greater than a force of the first biasing member (66).

19. A method of activating an autoinjector (10), in particular an autoinjector (10) in accordance with one of the preceding claims, the autoinjector (10) comprising an inner cap (12), an outer cap (32), an inner body (44), and an outer body (46), the method comprising the steps of: removing said inner cap (12) from a proximal end of said autoinjector (10);
pressing the outer cap (32) to move the inner body (44) relative to the outer body (46); and
fixing said inner body (44) to said outer body (46).
